# EUROPEAN PATENT APPLICATION

(11) **EP 4 663 759 A1**
(43) Date of publication of application: **17.12.2025**
(21) Application number: 25175813.2
(22) Date of filing: 12.05.2025
(51) Int. Cl.: C12N 15/70, C12N 9/04, C12N 9/08, G01N 27/327

(54) **BIOLOGICAL ENZYME MUTANT, METHOD FOR PREPARING THE SAME, AND USES THEREOF**

(30) Priority: 11.05.2024 CN 202410586985
(71) Applicant: Shanghai United Imaging Microelectronics Technology Co., Ltd., Shanghai 201899 (CN)
(72) Inventor: JIA, Jie, Shanghai, 201899 (CN)
(74) Representative: Murgitroyd & Company

(57) **Abstract**

Embodiments of the present disclosure provide a biological enzyme mutant, comprising a glucose oxidase mutant and/or a catalase mutant, wherein:
the glucose oxidase mutant comprises, relative to the wild-type glucose oxidase, at least one of the following sets of mutations:
(i) p.N43Q, p.N89Q, p.N161Q, p.N165Q, p.N258Q, p.N355Q, p.N388Q, and p.N473Q; or
(ii) p.N43A, p.N89A, p.N161A, p.N165A, p.N258A, p.N355A, p.N388A, and p.N473A;

when the biological enzyme mutant comprises a catalase mutant, the catalase mutant comprises, relative to the wild-type catalase, the mutations p.N148Q, p.N244Q, p.N439Q, and p.N481Q.

## Description

This application claims priority to Chinese Patent Application No. 202410586985.4, filed on May 11, 2024, the entire contents of which are hereby incorporated by reference.

### TECHNICAL FIELD

The present disclosure relates to biotechnology, and in particular, to a biological enzyme mutant, a method for preparing the same, and uses thereof.

### BACKGROUND

Due to the suboptimal electron transfer efficiency of biological enzymes currently employed in electrochemical sensors, researchers have undertaken extensive efforts to enhance the electron transfer efficiency. The first-generation electrochemical sensor utilizes hydrogen peroxide as an electron transfer mediator, characterized by slow electron transfer and susceptibility to interference. The second-generation electrochemical sensor incorporates an electron mediator, enabling fast electron transfer at low working voltages, and currently represents the mainstream technology. However, even with the addition of electron mediator, post-translational modifications such as phosphorylation, ubiquitination, and glycosylation frequently occur following the expression and purification of biological enzymes in host cells, attributed to the host's endogenous post-translational modification machinery. Among these, glycosylation, due to its large molecular weight, typically occurs on the surface of biological enzymes, posing significant steric hindrance to electron transfer.

Current approaches primarily involve in vitro deglycosylation of purified biological enzymes using enzymes such as N-glycoamidase F (PNGase F), peptide N-glycosidase A (PNGase A), endoglycosidase F (Endo F), or endoglycosidase H (Endo H). However, such methods often result in incomplete deglycosylation, necessitating improvements to traditional techniques.

Therefore, it is desirable to provide Biological Enzyme Mutant, Method for Preparing the Same, and Uses Thereof.

### SUMMARY

An aspect of the present disclosure may provide a method for preparing a biological enzyme mutant, including steps A and B;
Step A: Identify the glycosylation sites of the biological enzyme;
Step B: Mutate the glycosylation sites of the biological enzyme identified in Step A. Specifically, in Step A, the glycosylation modification sites are determined based on the protein structure and/or the protein gene sequence, and/or the glycoprotein modification sites are determined through a glycoprotein modification site prediction website.

In some embodiments, the aforementioned glycosylation modification sites comprise N - glycosylation modification sites and/or O - glycosylation modification sites. Further, the glycosylation groups comprise N - acetylglucosamine and/or mannose and/or fructose and/or galactose.

In some of these embodiments, the specific amino acid site for N - glycosylation modification is asparagine - X amino acid - serine/threonine, wherein the X-amino acid is any amino acid other than proline, and N - glycosylation modification occurs on the asparagine residue at this site.

In some of these embodiments, the O - glycosylated modified O - sugar chain is covalently linked to the free OH group of serine or threonine of the protein.

In some of these embodiments, the above - mentioned mutation methods comprise at least one of site - specific mutation methods and homologous recombination methods.

Specifically, the aforementioned mutation method involves mutating asparagine, the X amino acid, serine, and/or threonine in the N - glycosylation modification site asparagine - X amino acid - serine/threonine to one or more of the following amino acids: alanine, isoleucine, leucine, valine, methionine, phenylalanine, tryptophan, tyrosine, cysteine, glutamine, arginine, histidine, lysine, aspartate, glutamate, glycine, or proline. For the O - glycosylation modification site, serine and/or threonine are mutated to one or more of the following amino acids: alanine, isoleucine, leucine, valine, methionine, phenylalanine, tryptophan, tyrosine, asparagine, cysteine, glutamine, arginine, histidine, lysine, aspartate, glutamate, glycine, or proline.

Another aspect of the present disclosure may provide a biological enzyme mutant comprising at least one of a glucose oxidase mutant and a catalase mutant. The mutant glucose oxidase, compared to the wild - type glucose oxidase, has at least one of the following sets of mutations: Set 1: p.N43Q, p.N89Q, p.N161Q, p.N165Q, p.N258Q, p.N355Q, p.N388Q, and p.N473Q; Set 2: p.N43A, p.N89A, p.N161A, p.N165A, p.N258A, p.N355A, p.N388A, and p.N473A; when the biological enzyme mutant comprises a catalase mutant, the mutant catalase, compared to the wild - type catalase, has the mutations: p.N148Q, p.N244Q, p.N439Q, and p.N481Q; the amino acid sequence of the wild - type glucose oxidase is set forth in SEQ ID NO: 1, and the amino acid sequence of the wild - type catalase is set forth in SEQ ID NO: 2.

Specifically, the amino acid sequence shown as SEQ ID NO:1 is: MQTLLVSSLVVSLAAALPHYIRSNGIEASLLTDPKDVSGRTVDYIIAGGGLTGL TTAARLTENPNISVLVIESGSYESDRGPIIEDLNAYGDIFGSSVDHAYETVELA TNNQTALIRSGNGLGGSTLVNGGTWTRPHKAQVDSWETVFGNEGWNWDN VAAYSLQAERARAPNAKQIAAGHYFNASCHGVNGTVHAGPRDTGDDYSPIV KALMSAVEDRGVPTKKDFGCGDPHGVSMFPNTLHEDQVRSDAAREWLLPN YQRPNLQVLTGQYVGKVLLSQNGTTPRAVGVEFGTHKGNTHNVYAKHEVLL AAGSAVSPTILEYSGIGMKSILEPLGIDTVVDLPVGLNLQDQTTATVRSRITSA GAGQGQAAWFATFNETFGDYSEKAHELLNTKLEQWAEEAVARGGFHNTTA LLIQYENYRDWIVNHNVAYSELFLDTAGVASFDVWDLLPFTRGYVHILDKDPY LHHFAYDPQYFLNELDLLGQAAATQLARNISNSGAMQTYFAGETIPGDNLAY DADLSAWTEYIPYHFRPNYHGVGTCSMMPKEMGGVVDNAARVYGVQGLRV IDGSIPPTQMSSHVMTVFYAMALKISDAILEDYASMQ; the amino acid sequence shown as SEQ ID NO:2 is:

MADSRDPASDQMQHWKEQRAAQKADVLTTGAGNPVGDKLNVITVGPRGPL
LVQDVVFTDEMAHFDRERIPERVVHAKGAGAFGYFEVTHDITKYSKAKVFEHI
GKKTPIAVRFSTVAGESGSADTVRDPRGFAVKFYTEDGNWDLVGNNTPIFFI
RDPILFPSFIHSQKRNPQTHLKDPDMVWDFWSLRPESLHQVSFLFSDRGIPD
GHRHMNGYGSHTFKLVNANGEAVYCKFHYKTDQGIKNLSVEDAARLSQEDP
DYGIRDLFNAIATGKYPSWTFYIQVMTFNQAETFPFNPFDLTKVWPHKDYPLI
PVGKLVLNRNPVNYFAEVEQIAFDPSNMPPGIEASPDKMLQGRLFAYPDTHR
HRLGPNYLHIPVNCPYRARVANYQRDGPMCMQDNQGGAPNYYPNSFGAPE
QQPSALEHSIQYSGEVRRFNTANDDNVTQVRAFYVNVLNEEQRKRLCENIA
GHLKDAQIFIQKKAVKNFTEVHPDYGSHIQALLDKYNAEKPKNAIHTFVQSGS
HLAAREKANL.

In some embodiments, a glucose oxidase with an amino acid sequence set forth in SEQ ID NO:1 is mutated at p.N43Q, p.N89Q, p.N161Q, p.N165Q, p.N258Q, p.N355Q, p.N388Q, and p.N473Q to obtain a mutant with an amino acid sequence set forth in SEQ ID NO:3. Specifically, the amino acid sequence shown as SEQ ID NO:3

is:MQTLLVSSLVVSLAAALPHYIRSNGIEASLLTDPKDVSGRTVDYIIAGGGLT
GLTTAARLTENPQISVLVIESGSYESDRGPIIEDLNAYGDIFGSSVDHAYETVE
LATNQQTALIRSGNGLGGSTLVNGGTWTRPHKAQVDSWETVFGNEGWNW
DNVAAYSLQAERARAPNAKQIAAGHYFQASCHGVQGTVHAGPRDTGDDYS
PIVKALMSAVEDRGVPTKKDFGCGDPHGVSMFPNTLHEDQVRSDAAREWLL
PNYQRPNLQVLTGQYVGKVLLSQQGTTPRAVGVEFGTHKGNTHNVYAKHE
VLLAAGSAVSPTILEYSGIGMKSILEPLGIDTVVDLPVGLNLQDQTTATVRSRIT
SAGAGQGQAAWFATFQETFGDYSEKAHELLNTKLEQWAEEAVARGGFHQT
TALLIQYENYRDWIVNHNVAYSELFLDTAGVASFDVWDLLPFTRGYVHILDKD
PYLHHFAYDPQYFLNELDLLGQAAATQLARQISNSGAMQTYFAGETIPGDNL
AYDADLSAWTEYIPYHFRPNYHGVGTCSMMPKEMGGVVDNAARVYGVQGL
RVIDGSIPPTQMSSHVMTVFYAMALKISDAILEDYASMQ.

In some embodiments, a glucose oxidase with an amino acid sequence set forth in SEQ ID NO:1 is mutated at p.N43A, p.N89A, p.N161A, p.N165A, p.N258A, p.N355A, p.N388A, and p.N473A, resulting in a mutant with an amino acid sequence set forth in SEQ ID NO:4. Specifically, the amino acid sequence shown as SEQ ID NO:4

is:MQTLLVSSLVVSLAAALPHYIRSNGIEASLLTDPKDVSGRTVDYIIAGGGLT
GLTTAARLTENPAISVLVIESGSYESDRGPIIEDLNAYGDIFGSSVDHAYETVE
LATNAQTALIRSGNGLGGSTLVNGGTWTRPHKAQVDSWETVFGNEGWNW
DNVAAYSLQAERARAPNAKQIAAGHYFAASCHGVAGTVHAGPRDTGDDYSP
IVKALMSAVEDRGVPTKKDFGCGDPHGVSMFPNTLHEDQVRSDAAREWLLP
NYQRPNLQVLTGQYVGKVLLSQAGTTPRAVGVEFGTHKGNTHNVYAKHEVL
LAAGSAVSPTILEYSGIGMKSILEPLGIDTVVDLPVGLNLQDQTTATVRSRITS
AGAGQGQAAWFATFAETFGDYSEKAHELLNTKLEQWAEEAVARGGFHATT
ALLIQYENYRDWIVNHNVAYSELFLDTAGVASFDVWDLLPFTRGYVHILDKDP
YLHHFAYDPQYFLNELDLLGQAAATQLARAISNSGAMQTYFAGETIPGDNLA
YDADLSAWTEYIPYHFRPNYHGVGTCSMMPKEMGGVVDNAARVYGVQGLR
VIDGSIPPTQMSSHVMTVFYAMALKISDAILEDYASMQ.

In some embodiments, a catalase with an amino acid sequence set forth in SEQ ID NO:2 is mutated at p.N148Q, p.N244Q, p.N439Q, and p.N481Q to obtain a mutant with an amino acid sequence set forth in SEQ ID NO:5. Specifically, the amino acid sequence shown as SEQ ID NO:5

is:MADSRDPASDQMQHWKEQRAAQKADVLTTGAGNPVGDKLNVITVGPRG
PLLVQDVVFTDEMAHFDRERIPERVVHAKGAGAFGYFEVTHDITKYSKAKVF
EHIGKKTPIAVRFSTVAGESGSADTVRDPRGFAVKFYTEDGNWDLVGQNTPI
FFIRDPILFPSFIHSQKRNPQTHLKDPDMVWDFWSLRPESLHQVSFLFSDRGI
PDGHRHMNGYGSHTFKLVNANGEAVYCKFHYKTDQGIKQLSVEDAARLSQE
DPDYGIRDLFNAIATGKYPSWTFYIQVMTFNQAETFPFNPFDLTKVWPHKDY
PLIPVGKLVLNRNPVNYFAEVEQIAFDPSNMPPGIEASPDKMLQGRLFAYPDT
HRHRLGPNYLHIPVNCPYRARVANYQRDGPMCMQDNQGGAPNYYPNSFG
APEQQPSALEHSIQYSGEVRRFNTANDDQVTQVRAFYVNVLNEEQRKRLCE
NIAGHLKDAQIFIQKKAVKQFTEVHPDYGSHIQALLDKYNAEKPKNAIHTFVQS
GSHLAAREKANL.

Another aspect of the present disclosure may provide a nucleic acid for mediating the secretion and expression of the biological enzyme by a mutant in a host cell.

In some embodiments, the nucleic acid comprises a nucleic acid fragment encoding the amino acid sequence of a glucose oxidase mutant as set forth in SEQ ID NO: 3. Optionally, the nucleotide sequence of the nucleic acid encoding the amino acid sequence of the glucose oxidase mutant as set forth in SEQ ID NO: 3 is set forth in SEQ ID NO: 6. It is understood that, due to the degeneracy of codons, in other embodiments, the nucleotide sequence of the nucleic acid encoding the amino acid sequence of the glucose oxidase mutant as set forth in SEQ ID NO: 3 is not limited to the above - mentioned sequence but may also be other sequences. Specifically, the above nucleotide sequence shown as SEQ ID NO:6 is:

ATGCAGACTCTCCTTGTGAGCTCGCTTGTGGTCTCCCTCGCTGCGGCCC
TGCCACACTACATCAGGAGCAATGGCATTGAAGCCAGCCTCCTGACTGAT
CCCAAGGATGTCTCCGGCCGCACGGTCGACTACATCATCGCTGGTGGAG
GTCTGACTGGACTCACCACCGCTGCTCGTCTGACGGAGAACCCCCAAAT
CAGTGTGCTCGTCATCGAAAGTGGCTCCTACGAGTCGGACAGAGGTCCT
ATCATTGAGGACCTGAACGCCTACGGCGACATCTTTGGCAGCAGTGTAG
ACCACGCCTACGAGACCGTGGAGCTCGCTACCAACCAACAAACCGCGCT
GATCCGCTCCGGAAATGGTCTCGGTGGCTCTACTCTAGTGAATGGTGGC
ACCTGGACTCGCCCCCACAAGGCACAGGTTGACTCTTGGGAGACTGTCT
TTGGAAATGAGGGCTGGAACTGGGACAATGTGGCCGCCTACTCCCTCCA

GGCTGAGCGTGCTCGCGCACCAAATGCCAAACAGATCGCTGCTGGCCAC
TACTTCCAAGCATCCTGCCATGGTGTTCAAGGTACTGTCCATGCCGGACC
CCGCGACACCGGCGATGACTATTCTCCCATCGTCAAGGCTCTCATGAGC
GCTGTCGAAGACCGGGGCGTTCCCACCAAGAAAGACTTCGGATGCGGTG
ACCCCCATGGTGTGTCCATGTTCCCCAACACCTTGCACGAAGACCAATAT
GTTGGTAAGGTGCTCCTTAGCCAGCAAGGCACCACCCCTCGTGCCGTTG
GCGTGGAATTCGGCACCCACAAGGGCAACACCCACAACGTTTACGCTAA
GCACGAGGTCCTCCTGGCCGCGGGCTCCGCTGTCTCTCCCACAATCCTC
GAATATTCCGGTATCGGAATGAAGTCCATCCTGGAGCCCCTTGGTATCGA
CACCGTCGTTGACCTGCCCGTCGGCTTGAACCTGCAGGACCAGACCACC
GCTACCGTCCGCTCCCGCATCACCTCTGCTGGTGCAGGACAGGGACAG
GCCGCTTGGTTCGCCACCTTCCAAGAGACCTTTGGTGACTATTCCGAAAA
GGCACACGAGCTGCTCAACACCAAGCTGGAGCAGTGGGCCGAAGAGGC
CGTCGCCCGTGGCGGATTCCACCAAACCACCGCCTTGCTCATCCAGTAC
GAGAACTACCGCGACTGGATTGTCAACCACAACGTCGCGTACTCGGAAC
TCTTCCTCGACACTGCCGGAGTAGCCAGCTTCGATGTGTGGGACCTTCT
GCCCTTCACCCGAGGATACGTTCACATCCTCGACAAGGACCCCTACCTTC
ACCACTTCGCCTACGACCCTCAGTACTTCCTCAACGAGCTGGACCTGCTC
GGTCAGGCTGCCGCTACTCAACTGGCCCGCCAAATCTCCAACTCCGGTG
CCATGCAGACCTACTTCGCTGGGGAGACTATCCCCGGTGATAACCTCGC
GTATGATGCCGATTTGAGCGCCTGGACTGAGTACATCCCGTACCACTTCC
GTCCTAACTACCATGGCGTGGGTACTTGCTCCATGATGCCGAAGGAGAT
GGGCGGTGTTGTTGATAATGCTGCCCGTGTGTATGGTGTGCAGGGACTG
CGTGTCATTGATGGTTCTATTCCTCCTACGCAAATGTCGTCCCATGTCAT
GACGGTGTTCTATGCCATGGCGCTAAAAATTTCGGATGCTATCTTGGAAG
ATTATGCTTCCATGCAGTGA.

In some embodiments, the nucleic acid comprises a nucleic acid fragment encoding the amino acid sequence of a glucose oxidase mutant as set forth in SEQ ID NO: 4. Optionally, the nucleotide sequence of the nucleic acid encoding the amino acid sequence of the glucose oxidase mutant as set forth in SEQ ID NO: 4 is set forth in SEQ ID NO: 7. It is understood that, due to the degeneracy of codons, in other embodiments, the nucleotide sequence of the nucleic acid encoding the amino acid sequence of the glucose oxidase mutant as set forth in SEQ ID NO: 4 is not limited to the above - mentioned sequence but may also be other sequences. Specifically, the above nucleotide sequence shown as SEQ ID NO:7 is:

ATGCAGACTCTCCTTGTGAGCTCGCTTGTGGTCTCCCTCGCTGCGGCCC
TGCCACACTACATCAGGAGCAATGGCATTGAAGCCAGCCTCCTGACTGAT
CCCAAGGATGTCTCCGGCCGCACGGTCGACTACATCATCGCTGGTGGAG
GTCTGACTGGACTCACCACCGCTGCTCGTCTGACGGAGAACCCCGCCAT
CAGTGTGCTCGTCATCGAAAGTGGCTCCTACGAGTCGGACAGAGGTCCT
ATCATTGAGGACCTGAACGCCTACGGCGACATCTTTGGCAGCAGTGTAG
ACCACGCCTACGAGACCGTGGAGCTCGCTACCAACGCCCAAACCGCGCT
GATCCGCTCCGGAAATGGTCTCGGTGGCTCTACTCTAGTGAATGGTGGC
ACCTGGACTCGCCCCCACAAGGCACAGGTTGACTCTTGGGAGACTGTCT
TTGGAAATGAGGGCTGGAACTGGGACAATGTGGCCGCCTACTCCCTCCA
GGCTGAGCGTGCTCGCGCACCAAATGCCAAACAGATCGCTGCTGGCCAC
TACTTCGCCGCATCCTGCCATGGTGTTGCCGGTACTGTCCATGCCGGAC
CCCGCGACACCGGCGATGACTATTCTCCCATCGTCAAGGCTCTCATGAG
CGCTGTCGAAGACCGGGGCGTTCCCACCAAGAAAGACTTCGGATGCGGT
GACCCCCATGGTGTGTCCATGTTCCCCAACACCTTGCACGAAGACCAATA
TGTTGGTAAGGTGCTCCTTAGCCAGGCCGGCACCACCCCTCGTGCCGTT
GGCGTGGAATTCGGCACCCACAAGGGCAACACCCACAACGTTTACGCTA
AGCACGAGGTCCTCCTGGCCGCGGGCTCCGCTGTCTCTCCCACAATCCT
CGAATATTCCGGTATCGGAATGAAGTCCATCCTGGAGCCCCTTGGTATCG
ACACCGTCGTTGACCTGCCCGTCGGCTTGAACCTGCAGGACCAGACCAC
CGCTACCGTCCGCTCCCGCATCACCTCTGCTGGTGCAGGACAGGGACAG
GCCGCTTGGTTCGCCACCTTCGCCGAGACCTTTGGTGACTATTCCGAAAA
GGCACACGAGCTGCTCAACACCAAGCTGGAGCAGTGGGCCGAAGAGGC
CGTCGCCCGTGGCGGATTCCACGCCACCACCGCCTTGCTCATCCAGTAC
GAGAACTACCGCGACTGGATTGTCAACCACAACGTCGCGTACTCGGAAC
TCTTCCTCGACACTGCCGGAGTAGCCAGCTTCGATGTGTGGGACCTTCT
GCCCTTCACCCGAGGATACGTTCACATCCTCGACAAGGACCCCTACCTTC
ACCACTTCGCCTACGACCCTCAGTACTTCCTCAACGAGCTGGACCTGCTC
GGTCAGGCTGCCGCTACTCAACTGGCCCGCGCCATCTCCAACTCCGGTG
CCATGCAGACCTACTTCGCTGGGGAGACTATCCCCGGTGATAACCTCGC
GTATGATGCCGATTTGAGCGCCTGGACTGAGTACATCCCGTACCACTTCC
GTCCTAACTACCATGGCGTGGGTACTTGCTCCATGATGCCGAAGGAGAT
GGGCGGTGTTGTTGATAATGCTGCCCGTGTGTATGGTGTGCAGGGACTG
CGTGTCATTGATGGTTCTATTCCTCCTACGCAAATGTCGTCCCATGTCAT

GACGGTGTTCTATGCCATGGCGCTAAAAATTTCGGATGCTATCTTGGAAG
ATTATGCTTCCATGCAGTGA.

In some embodiments, the nucleic acid comprises a nucleic acid fragment encoding the amino acid sequence of a glucose oxidase mutant as set forth in SEQ ID NO: 5. Optionally, the nucleotide sequence of the nucleic acid encoding the amino acid sequence of the glucose oxidase mutant as set forth in SEQ ID NO: 5 is set forth in SEQ ID NO: 8. It is understood that, due to the degeneracy of codons, in other embodiments, the nucleotide sequence of the nucleic acid encoding the amino acid sequence of the glucose oxidase mutant as set forth in SEQ ID NO: 5 is not limited to the above - mentioned sequence but may also be other sequences. Specifically, the above nucleotide sequence shown as SEQ **ID** NO:8 is:

ATGGCTGACAGCCGGGATCCCGCCAGCGACCAGATGCAGCACTGGAAG
GAGCAGCGGGCCGCGCAGAAAGCTGATGTCCTGACCACTGGAGCTGGT
AACCCAGTAGGAGACAAACTTAATGTTATTACAGTAGGGCCCCGTGGGC
CCCTTCTTGTTCAGGATGTGGTTTTCACTGATGAAATGGCTCATTTTGACC
GAGAGAGAATTCCTGAGAGAGTTGTGCATGCTAAAGGAGCAGGGGCCTT
TGGCTACTTTGAGGTCACACATGACATTACCAAATACTCCAAGGCAAAGG
TATTTGAGCATATTGGAAAGAAGACTCCCATCGCAGTTCGGTTCTCCACT
GTTGCTGGAGAATCGGGTTCAGCTGACACAGTTCGGGACCCTCGTGGGT
TTGCAGTGAAATTTTACACAGAAGATGGTAACTGGGATCTCGTTGGACAA
AACACCCCCATTTTCTTCATCAGGGATCCCATATTGTTTCCATCTTTTATC
CACAGCCAAAAGAGAAATCCTCAGACACATCTGAAGGATCCGGACATGG
TCTGGGACTTCTGGAGCCTACGTCCTGAGTCTCTGCATCAGGTTTCTTTC
TTGTTCAGTGATCGGGGGATTCCAGATGGACATCGCCACATGAATGGATA
TGGATCACATACTTTCAAGCTGGTTAATGCAAATGGGGAGGCAGTTTATT
GCAAATTCCATTATAAGACTGACCAGGGCATCAAACAACTTTCTGTTGAA
GATGCGGCGAGACTTTCCCAGGAAGATCCTGACTATGGCATCCGGGATC
TTTTTAACGCCATTGCCACAGGAAAGTACCCCTCCTGGACTTTTTACATCC
AGGTCATGACATTTAATCAGGCAGAAACTTTTCCATTTAATCCATTCGATC
TCACCAAGGTTTGGCCTCACAAGGACTACCCTCTCATCCCAGTTGGTAAA
CTGGTCTTAAACCGGAATCCAGTTAATTACTTTGCTGAGGTTGAACAGATA
GCCTTCGACCCAAGCAACATGCCACCTGGCATTGAGGCCAGTCCTGACA
AAATGCTTCAGGGCCGCCTTTTTGCCTATCCTGACACTCACCGCCATCGC
CTGGGACCCAATTATCTTCATATACCTGTGAACTGTCCCTACCGTGCTCG

AGTGGCCAACTACCAGCGTGACGGCCCGATGTGCATGCAGGACAATCAG
GGTGGTGCTCCAAATTACTACCCCAACAGCTTTGGTGCTCCGGAACAACA
GCCTTCTGCCCTGGAGCACAGCATCCAATATTCTGGAGAAGTGCGGAGA
TTCAACACTGCCAATGATGATCAAGTTACTCAGGTGCGGGCATTCTATGT
GAACGTGCTGAATGAGGAACAGAGGAAACGTCTGTGTGAGAACATTGCC
GGCCACCTGAAGGATGCACAAATTTTCATCCAGAAGAAAGCGGTCAAGC
AATTCACTGAGGTCCACCCTGACTACGGGAGCCACATCCAGGCTCTTCT
GGACAAGTACAATGCTGAGAAGCCTAAGAATGCGATTCACACCTTTGTGC
AGTCCGGATCTCACTTGGCGGCAAGGGAGAAGGCAAATCTGTGA.

In some embodiments, the nucleic acid of any of the above embodiments also comprises transcriptional elements, such as a promoter and a terminator. It is understood that in some embodiments, the nucleic acid of any of the above embodiments may not comprise transcriptional elements. In this case, when in use, the said nucleic acid can be inserted into a vector with the corresponding transcriptional element for expression.

In some of these embodiments, the nucleic acid may be incorporated into a recombinant vector to facilitate the secretion and expression of a biological enzyme mutant.

An embodiment of the present application also provides a recombinant vector containing a nucleic acid encoding the biological enzyme mutant. The nucleic acid includes a fragment that encodes the amino acid sequence of a glucose oxidase mutant as set forth in SEQ ID NO: 3.

In some embodiments, the vector contains the nucleic acid of any of the above embodiments, where the encoded amino acid sequence is that of a glucose oxidase mutant as set forth in SEQ ID NO: 4.

In some embodiments, the vector contains the nucleic acid of any of the above embodiments, where the encoded amino acid sequence is that of a catalase mutant as set forth in SEQ ID NO: 5.

In one optional specific example, the vector used to express the enzyme mutant is pMJB1. Point mutation primers are designed, and site-directed mutagenesis PCR is performed to obtain a target fragment. The target sequence of the enzyme mutant is cloned into a restriction enzyme site of DPN1, and the expression vector containing the enzyme is transformed or transfected into a host cell to express the target protein.

Another aspect of the present disclosure may provide a host cell comprising a recombinant expression vector in its genome, which is capable of producing a biological enzyme mutant.

In some of these embodiments, the host cell is transformed with a recombinant vector comprising any of the nucleic acids described in the above embodiments: a nucleic acid fragment encoding the amino acid sequence of a glucose oxidase mutant as set forth in SEQ ID NO: 3 or SEQ ID NO: 4; and a nucleic acid fragment encoding the amino acid sequence of a catalase mutant as set forth in SEQ ID NO: 5.

In some of these embodiments, the host cell is a cell that clones the nucleic acid fragment encoding the biological enzyme mutant.

In some of these embodiments, the host cell is a cell that expresses the nucleic acid fragment encoding the biological enzyme mutant..

In some of these embodiments, the host cell is a recipient cell, and the nucleic acid fragment encoding the biological enzyme mutant or the recombinant vector is contained within the recipient cell..

In some of these embodiments, the recipient cell is selected from the group comprising *Escherichia coli, Agrobacterium, Saccharomyces cerevisiae, Pichia pastoris, Aspergillus niger,* an animal cell, and a plant cell. In particular, the recipient cell may be *Escherichia coli* DH5α, *Escherichia coli* Top10, *Escherichia coli* Origami(DE3), *Agrobacterium* AGL1, *Aspergillus niger, Pichia pastoris* GS115, or *Pichia pastoris* SMD1168. The recipient cells are not limited to those explicitly listed, as other commonly used recipient cells in the art may also act as host cells.

Another aspect of the present disclosure may provide an application of the said biological enzyme mutant, the said nucleic acid, the said recombinant vector, or the said host cell in preparing the biological enzyme.

Another aspect of the present disclosure may provide a method for preparing a biological enzyme mutant, which comprises: introducing mutations into a least one of (i) the wild-type glucose oxidase having the amino acid sequence of SEQ ID NO:1 or (ii) the wild-type catalase having the amino acid sequence of SEQ ID NO:2, wherein: for the glucose oxidase, introducing mutations at positions 43, 89, 161, 165, 258, 355, 388, and 473 to produce either (A) asparagine-to-glutamine substitutions (N→Q) or (B) asparagine-to-alanine substitutions (N→A); and, when introducing mutations into the wild-type catalase, for the catalase, introducing mutations at positions 148, 244, 439, and 481 to produce asparagine-to-glutamine substitutions (N→Q).

In some of these embodiments, a method for preparing a biological enzyme mutant, which comprises: steps S100 to S300. Step S100: preparing the first - linear recombinant vector and/or the second - linear recombinant vector ,respectively the first - linear recombinant vector includes the glucose oxidase gene, and the second - linear recombinant vector includes the catalase gene.Step S200: using primers with sequences set forth in SEQ ID NO:13 - 28 , perform the first PCR amplification on the first - linear recombinant vector, and obtain the first mutant recombinant vector . Alternatively, using primers with sequences set forth in SEQ ID NO:29 - 44, to perform the first PCR amplification on the first - linear recombinant vector, and obtain the second mutant recombinant vector. Additionally or alternatively, using primers set forth in SEQ ID NO: 49 - 56 to perform the second PCR amplification on the second - linear recombinant vector, and obtain the third mutant recombinant vector. Step S300: transforming the first mutant recombinant vector, or the second mutant recombinant vector, and/or the third mutant recombinant vector into host cells respectively, and culture the host cells to prepare the enzyme mutant.

In some of these embodiments, step S100 comprises steps S110 to S120. Step S110: Using primers set forth in SEQ ID NO: 9 - 10 to amplify the above - mentioned glucose oxidase by PCR, and obtain the target fragment of glucose oxidase. Additionally or alternatively, using primers set forth in SEQ ID NO: 45 - 46 respectively to amplify the above - mentioned catalase by PCR, and obtain the target fragment of catalase. Steps 120:Mixing the glucose oxidase target fragment and/or catalase target fragment with linearized ligation vectors, respectively. Ligating the target fragments with the ligation vectors to obtain the first-line recombinant vector and/or the second-line recombinant vector.

In some of these embodiments, each 20µL reaction system comprises 1µL to 5µL of the glucose oxidase target fragment and 1µL to 5µL of the linearized ligation vector.

In some of these embodiments, each 20µL reaction system comprises 1µL to 5µL of the catalase - targeted fragment and 1µL to 5µL of the linearized ligation vector.

In some of these embodiments, step S120 includes steps S121 to S122. Specifically, Step S121: Mixing the glucose oxidase target fragment with linearized ligation vectors and recombinases. Incubating the mixtures at 37°C for at least 30 minutes to obtain the first ligation product. Alternatively or additionally, mixing the catalase target fragment with the linearized ligation vector and recombinase. Incubating this mixture at 37°C for at least 30 minutes to get a second ligation product. Step S122: Subject the first ligation product to heat - shock. Transfer it into competent host cells. Screen for monoclonal positive recombinants to obtain the first - linear recombinant vector. Alternatively or additionally, subject the second ligation product to heat - shock. Transfer it into competent host cells. Screen for monoclonal positive recombinants to obtain the second - linear recombinant vector.

In some of these embodiments, the recombinant enzyme is Exnase II.

In some of these embodiments, the heat - shock temperature is 40°C to 45°C, and the heat - shock time is 40 seconds to 50 seconds.

In some of these embodiments, the heat - shock temperature is 42°C and the heat - shock time is 45 seconds.

In some of these embodiments, the host cell is *Escherichia coli* DH5α.

In some of these embodiments, step S200 includes steps S210 to S230. Specifically, Step S210: Use primers with sequences set forth in SEQ ID NO:13 - 28 and SEQ ID NO:29 - 44 to perform the first PCR amplification of the first - linear recombinant vector, thereby obtaining the first amplification product and the second amplification product. Use primers set forth in SEQ ID NO: 49 - 56 to perform the second PCR on the second - linear recombinant vector, and obtain the third amplification product; Step S220: Mix the first amplification product, the second amplification product, and the third amplification product with restriction endonucleases respectively, and incubate the mixtures at 37°C for at least 60 minutes to obtain the first, second, and third digested products respectively; Step S230: Subject the first, second, and third digested products to heat - shock treatment into receptive host cells, and screen for monoclonal positive recombinants to obtain the first mutant recombinant vector, the second mutant recombinant vector, and the third mutant recombinant vector.

In some of these embodiments, the procedure of the first PCR amplification reaction described above is set as follows: pre - denaturation at 95°C for 3 to 5 minutes; denaturation at 94°C for 10 to 30 seconds, annealing at 56°C to 60°C for 10 to 30 seconds, extension at 72°C for 1 to 5 minutes, for a total of 30 to 35 cycles; final extension at 72°C for 3 to 7 minutes, followed by storage of the PCR-amplified products at 4°C.It is understood that in some other specific examples, the PCR procedure may be reasonably adjusted.

In some of these embodiments, the first PCR amplification procedure is set to: pre - denaturation at 95°C for 3 minutes; denaturation at 94°C for 15 seconds, annealing at 60°C for 15 seconds, extension at 72°C for 3 minutes, with a total of 30 cycles; final extension at 72°C for 5 minutes, followed by storage of the PCR products at 4°C.

In some embodiments, the procedure for the second PCR amplification reaction described above is set as follows: pre - denaturation at 95°C for 3 to 5 minutes; denaturation at 94°C for 10 to 30 seconds, annealing at 56°C to 60°C for 10 to 30 seconds, extension at 72°C for 1 to 5 minutes, for a total of 30 to 35 cycles; final extension at 72°C for 3 to 7 minutes, followed by storage of the PCR - amplified products at 4°C. It is understood that in some other specific examples, the PCR procedure can be reasonably adjusted.

In some of these embodiments, the amplification procedure of the second PCR is set to: pre - denaturation at 95°C for 3 minutes; denaturation at 94°C for 15 seconds, annealing at 60°C for 15 seconds, extension at 72°C for 3 minutes, for a total of 30 cycles; final extension at 72°C for 5 minutes, followed by storage of the PCR products at 4°C.

In some of these embodiments, the restriction endonuclease is DPN1.

In some of these embodiments, the heat - shock temperature is 40°C to 45°C, and the heat - shock time is 40 seconds to 50 seconds.

In some of these embodiments, the heat - shock temperature is 42°C and the heat - shock time is 45 seconds.

In some of these embodiments, the host cell is *Escherichia coli* DH5α.

In some of these embodiments, the first mutant recombinant vector, the second mutant recombinant vector, and/or the third mutant recombinant vector are respectively introduced into *Agrobacterium* for co - transformation culture with *Aspergillus niger.* Subsequently, the transformed *Aspergillus niger* is subjected to fermentation culture to prepare the biological enzyme mutant.

In some of these embodiments, the above fermentation temperature is 30°C to 35°C.

In some of these embodiments, the said fermentation time is 3 days to 8 days.

In some of these embodiments, the transformed host cells are subjected to fermentation culture. The fermentation products are collected to prepare the primary products containing the biological enzyme mutants. Then, the primary products are purified to obtain the purified biological enzyme mutant.

Another aspect of the present disclosure may provide an application of the biological enzyme mutant in the preparation of a biosensing element comprising a bioenzyme electrode or a biosensor.

Another aspect of the present disclosure may provide a bioenzyme electrode. The bioenzyme electrode comprises a substrate electrode, and the substrate electrode is loaded with a modification material; the modification material comprises one of the biological enzyme mutants described above.

In some of these embodiments, the substrate electrode is selected from the group consisting of at least one of glassy carbon electrodes, gold electrodes, graphite electrodes, and carbon paste electrodes.

Another aspect of the present disclosure may provide a biosensor comprising the bioenzyme electrode.

### BRIEF DESCRIPTION OF THE DRAWINGS

The present disclosure is further illustrated in terms of exemplary embodiments. These exemplary embodiments are described in detail with reference to according to the drawings. These embodiments are non-limiting exemplary embodiments, in which like reference numerals represent similar structures, and wherein:
FIG. 1: Electrophoresis of DNA fragments for wild-type and deglycosylated glucose oxidase in Example 1.
FIG. 2: SDS-PAGE analysis of wild-type and deglycosylated glucose oxidase in Example 1.
FIG. 3: Redox potential detection results for wild-type and deglycosylated glucose oxidase in Example 1 (x-axis: voltage; y-axis: current).
FIG. 4: Current response to glucose concentration for wild-type and deglycosylated glucose oxidase on a bioelectrochemical sensor in Example 1 (x-axis: time; y-axis: current).
FIG. 5: Linear correlation between current and glucose concentration for wild-type and deglycosylated glucose oxidase in Example 1 (x-axis: concentration; y-axis: current).
FIG. 6: Redox potential detection results for wild-type and deglycosylated glucose oxidase in Example 2 (x-axis: voltage; y-axis: current).
FIG. 7: Electrophoresis of DNA fragments for wild-type and deglycosylated catalase in Example 3.
FIG. 8: SDS-PAGE analysis of wild-type and deglycosylated catalase in Example 3.
FIG. 9: Redox potential detection results for wild-type and deglycosylated catalase in Example 3 (x-axis: voltage; y-axis: current).
FIG. 10: Current response to glucose concentration for wild-type and deglycosylated catalase on a bioelectrochemical sensor in Example 3 (x-axis: time; y-axis: current).
FIG. 11: Linear correlation between current and glucose concentration for wild-type and deglycosylated catalase in Example 3 (x-axis: concentration; y-axis: current).
FIG. 12: Redox potential detection results for wild-type and deglycosylated glucose oxidase in Example 4(Proportional Example 1) (x-axis: voltage; y-axis: current).
FIG. 13: Redox potential detection results for wild-type and deglycosylated glucose oxidase in Example 5(Proportional Example 2) (x-axis: voltage; y-axis: current).
FIG. 14: Redox potential detection results for wild-type and deglycosylated catalase in Example 6(Proportional Example 3) (x-axis: voltage; y-axis: current).
FIG. 15: Redox potential detection results for wild-type and deglycosylated catalase in Example 7(Proportional Example 4) (x-axis: voltage; y-axis: current).

### DETAILED DESCRIPTION

To facilitate the understanding of this application, a more comprehensive description of this application will be provided below. However, this application can be implemented in various forms and is not limited to the embodiments described herein. Instead, these embodiments are provided to make the disclosure of this application more thorough.

Unless otherwise defined, all technical and scientific terms used herein have the same meanings as commonly understood by a person skilled in the art to which this application pertains. The terms used in the specification of this application are solely for describing specific embodiments and are not intended to limit this application.

The terms "includes", "contains", or any other variations thereof are intended to cover non - exclusive inclusion. Thus, a process, method, article, or apparatus comprising a set of elements includes not only those elements but also other elements not explicitly listed or inherent to such process, method, article, or apparatus. Without further limitation, the term "includes an element of..." does not preclude the existence of additional identical elements in a process, method, article, or device that includes the element. The indefinite articles "a" and "an" before an element or component in this application do not restrict the quantitative requirements (i.e., the number of occurrences) of the element or component. Therefore, the words "one" or "a" should be construed to include one or at least one, and elements or components in the singular also include the plural form, unless it is clear that the quantities mentioned refer only to the singular form. "Many" means at least two, such as two, three, etc., unless otherwise explicitly and specifically qualified.

Enzymes are biological macromolecules, such as proteins and/or nucleic acids, with catalytic functions, generally including redox enzymes, transferases, hydrolases, lyases, isomerases, and/or synthases.

Currently, biological enzymes used in electrochemical sensors have low electron - transfer efficiency, which has spurred extensive research to improve this efficiency. The first - generation electrochemical sensors use hydrogen peroxide as an electron - transfer mediator. They are characterized by slow electron transfer and are susceptible to interference. The second - generation sensors incorporate electron mediator, enabling faster electron transfer at lower working voltages, and they are currently the mainstream. However, even with electron mediator, post - translational modifications such as phosphorylation, ubiquitination, and glycosylation often occur after biological enzymes are expressed and purified in host cells because of the host's endogenous modification machinery. Among these, glycosylation, due to its large molecular weight, typically occurs on the surface of biological enzymes, creating significant steric hindrance to electron transfer. Current approaches mainly involve in vitro deglycosylation of purified biological enzymes using enzymes such as N - glycoamidase F (PNGase F), peptide N - glycosidase A (PNGase A), endoglycosidase F (Endo F), or endoglycosidase H (Endo H). Nevertheless, such methods often lead to incomplete deglycosylation, indicating the need for improvements to traditional techniques. The present application provides a mutant protein with enhanced deglycosylation efficiency through protein engineering. By transforming mutant genes into host cells to directly express and purify deglycosylated biological enzymes, the efficiency and yield of deglycosylation are improved. The deglycosylated biological enzyme mutant has shorter electron - transfer distances, which enhances electron - transfer efficiency and facilitates its application in biosensors.

The following is a detailed description in conjunction with specific embodiments. Unless otherwise specified, the following embodiments do not include components other than unavoidable impurities. The reagents and instruments used in the embodiments, unless otherwise specified, are the common choices in the field. The experimental methods not specified in the embodiments shall be implemented in accordance with conventional conditions, such as those described in the literature or books or the methods recommended by the manufacturer.

### Example 1

### 1. Preparation of Recombinant Expression Plasmid pMJB1-GOx

Using a glucose oxidase-producing bacterium as the template, the glucose oxidase gene (GOx) fragment was amplified by PCR using primer sequences 5'- ATGCAGACTCTCCTTGTGAGC -3' (SEQ ID NO:9) and 5'-TCACTGCATGGAAGCATAATCTT -3'(SEQ ID NO:10). Target bands and heterobands were separated by DNA agarose gel electrophoresis, and the glucose oxidase gene solution was obtained after gel recovery.

Linearized forward primer (5'-ATCCATACGATGTTCCTGATTATGC-3', SEQ ID NO:11) and reverse primer (5'-ATACTGTTTAAATTAATCTATGCTAGCTTATTT-3', SEQ ID NO:12) were designed. Following linearization of the pMJB1 vector via homologous recombination, the glucose oxidase gene solution was inserted to generate the pMJB1-GOx expression plasmid.

The plasmid was linearized by PCR using commercialized PrimerSTAR Max Premix (2X). The PCR reaction solution was prepared as shown in in the following table.

**Table 1**

| **Reagents** | **Amount Used (µL)** |
|---|---|
| PrimeSTAR Max Premix (2X) | 15 |
| Forward Primer | 1 |
| Reverse Primer | 1 |
| pMJB1 Template | 1 |
| Sterilized Water | 12 |

The prepared PCR reaction system was placed in a PCR apparatus and amplified according to the procedure in Table 2.

**Table 2**

| **Temperature (°C)** | **Time** | **Number of Cycles** |
|---|---|---|
| 95 | 3 min | 0 |
| 95 | 15 s | 30 |
| 60 | 15 s | |
| 72 | 3 min | |
| 72 | 5 min | 0 |
| 4 | Hold | 0 |

Following the PCR reaction, the reaction solution was electrophoresed through a DNA agarose gel, and the gel was recovered to obtain the linearized pMJB1 vector.

### 2. Construction of Recombinant Expression Plasmid pMJB1-GOx

The linearized pMJB1 vector was ligated with the glucose oxidase gene fragment using Exnase II recombinase, with the reaction setup shown in Table 3, The reaction solution was incubated at 37°C for 30 minutes, then immediately cooled to 4°C for storage.

**Table 3**

| **Reagents** | **Amount Used (µL)** |
|---|---|
| 5X CE II Buffer | 4 |
| Exnase II | 2 |
| Glucose Oxidase Gene | 4 |
| Linearized pMJB1 | 2 |
| Sterilized Water | 8 |

The pMJB1-GOx recombinant products were transformed into DH5α competent cells via heat shock. Add products to ice-cold competent cells, incubate on ice for 30 minutes. Heat shock at 42°C for 45 seconds, then return to ice for 2-3 minutes. Resuspend in 1 mL non-resistant LB liquid medium, culture at 37°C with shaking for 1 hour. Centrifuge at 2000 rpm, discard the supernatant, resuspend the bacterial pellet in residual medium, and plate on LB agar containing ampicillin. After overnight incubation at 37°C, the plate was sent for sequencing to confirm the successfully constructed pMJB1-GOx expression plasmid.

### 3. Construction of Recombinant Mutant Expression Plasmid pMJB1-GOx-deglycosylation

Using the pMJB1-GOx plasmid as a template, asparagine residues at positions N43, N89, N161, N165, N258, N355, N388, and N473 were mutated to glutamine (Q) using the point mutation primers listed in Table 4.

**Table 4**

| | **Forward Primers (5'-3')** | **Reverse Primers (5'-3')** |
|---|---|---|
| N43 Q | GAACCCCCAATCAGTGTGCTCGT | ACACTGATTGGGGGTTCTCCG |
| | CATCGAAAG (SEQ ID NO:13) | TCAGACGAGCA (SEQ ID NO:14) |
| N89 Q | TACCAACCAACAAACCGCGCTGA TCCGCTCCG (SEQ ID NO:15) | CGGTTTGTTGGTTGGTAGCGA GCTCCACGGTC (SEQ ID NO:16) |
| N16 1Q | CTACTTCCAAGCATCCTGCCATG GTGTTAATG (SEQ ID NO:17) | AGGATGCTTGGAAGTAGTGGC CAGCAGCGATC (SEQ ID NO:18) |
| N16 5Q | TGGTGTTCAAGGTACTGTCCATG CCGGACCCC (SEQ ID NO:19) | CAGTACCTTGAACACCATGGC AGGATGCGTTG (SEQ ID NO:20) |
| N25 8Q | TGCTCCTTAGCCAGCAAGGCAC CACCCCTCGTGCC (SEQ ID NO:21) | TTGCTGGCTAAGGAGCACCTT ACCAACATATT (SEQ ID NO:22) |
| N35 5Q | CACCTTCCAAGAGACCTTTGGTG ACTATTCCGA (SEQ ID NO:23) | AGGTCTCTTGGAAGGTGGCGA ACCAAGCGGCC (SEQ ID NO:24) |
| N38 8Q | ATTCCACCAAACCACCGCCTTGC TCATCCAGT (SEQ ID NO:25) | CGGTGGTTTGGTGGAATCCGC CACGGGCGACG (SEQ ID |
| | | NO:26) |
| N47 3Q | CCGCCAAATCTCCAACTCCGGT GCCATGCAGA (SEQ ID NO:27) | AGTTGGAGATTTGGCGGGCCA GTTGAGTAGCG (SEQ ID NO:28) |

PCR was performed using PrimerStar Max Premix (2X), with the reaction solution prepared as shown in Table 5.

**Table 5**

| **Reagents** | **Amount Used (µL)** |
|---|---|
| PrimeSTAR Max Premix (2X) | 10 |
| Forward Primer | 1 |
| Reverse Primer | 1 |
| Gene Template | 1 |
| Sterilized Water | 7 |

The PCR amplification procedure followed Table 6.

**Table 6**

| **Temperature (°C)** | **Time** | **Number of Cycles** |
|---|---|---|
| 95 | 3 min | 0 |
| 95 | 15 s | 30 |
| 60 | 15 s | |
| 72 | 3 min | |
| 72 | 5 min | 0 |
| 4 | Hold | 0 |

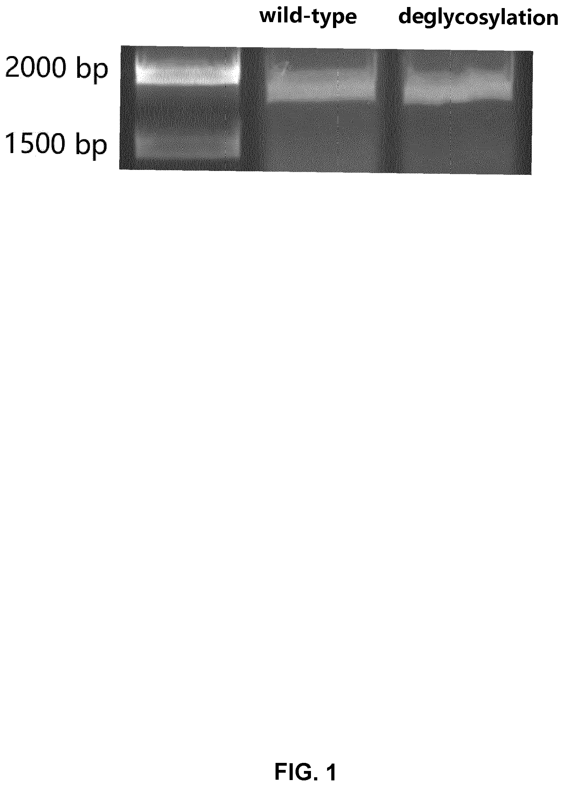

After PCR, 1 µL of DPN1 restriction endonuclease was added, and the reaction was incubated at 37°C for 1 hour. The products were cooled to 4°C and transformed into DH5α competent cells using the same heat shock method as above. Transformed cells were plated on LB agar with ampicillin, and the resulting plasmid, pMJB1-GOx-deglycosylation, was confirmed by sequencing. DNA agarose gel electrophoresis yielded wild-type and deglycosylated glucose oxidase fragments, as shown in Figure 1.

### 4. Production of Wild-Type and Mutant Glucose Oxidase

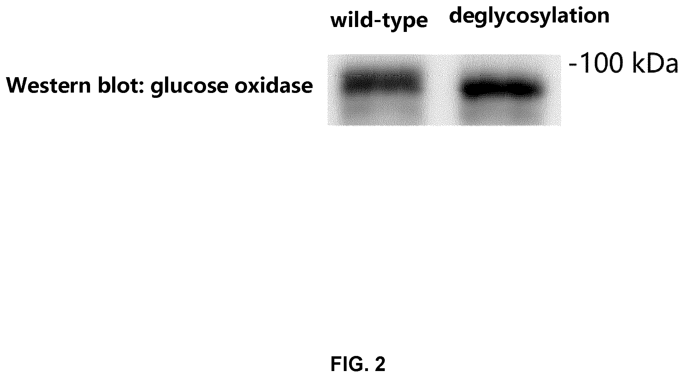

The constructed plasmid was transformed into *Agrobacterium* AGL1 via the freeze-thaw method. Correct transformants were selected and activated in liquid YEB medium. Fifty microliters each of activated *Agrobacterium* AGL1 and *Aspergillus niger* mycelium (from liquid potato medium, high glucoamylase yield) were co-coated on a PDA plate containing 200 µmol/L acetosyringone (AS) and covered with cellophane, then co-cultured at 28°C for 3 days. The cellophane was transferred to a PDA plate with 250 mg/L hygromycin B and 400 mg/L cefotaxime sodium for 1 day, after which the cellophane was removed, and culture continued at 32°C for 7-10 days until *Aspergillus niger* growth was evident. Fermentation products were purified to obtain wild-type glucose oxidase (GOx) and deglycosylated mutant (GOX-deglycosylation) pure enzymes. Western blot analysis verified deglycosylation and protein expression, as shown in Figure 2.

### 5. Redox Potential Detection

Ferrocenyl methanol served as an electron mediator for wild-type/deglycosylated glucose oxidase.

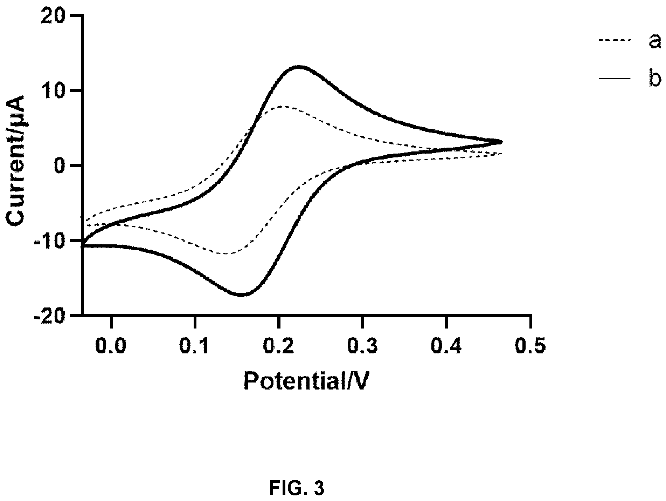

Polish and ultrasonically clean a glassy carbon electrode. Add 5 µL ferrocenyl methanol solution to the electrode surface, followed by drops of wild-type/mutant enzyme solution. Cure the electrode in a glutaraldehyde vapor environment for 30 minutes, then dry overnight at 45°C.Measure redox potential using an Ag/AgCl reference electrode and platinum counter electrode in a phosphate buffer system via an electrochemical workstation, as shown in Figure 3.

### 6. Electrochemical Performance Testing with Glucose Buffers

Screen-print conductive paste layer-by-layer onto a substrate, cure, print insulating medium paste between layers, and laser-cut and clean to form the substrate electrode (working electrode and counter electrode: platinum carbon paste; reference electrode: silver/silver chloride paste). Dissolve the wild-type/deglycosylated glucose oxidase obtained above in the BSA solution, and immerse the substrate electrode-cobalt ion complex in a BSA solution containing glucose oxidase and glutaraldehyde for 10 minutes, then stand at room temperature for 1 hour to obtain the glucose oxidase-coated sensor electrode.

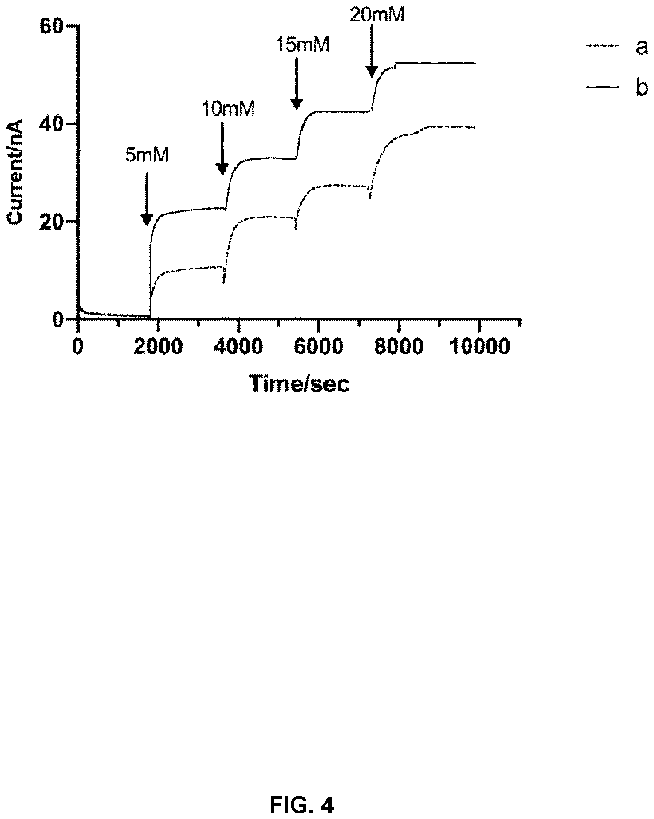

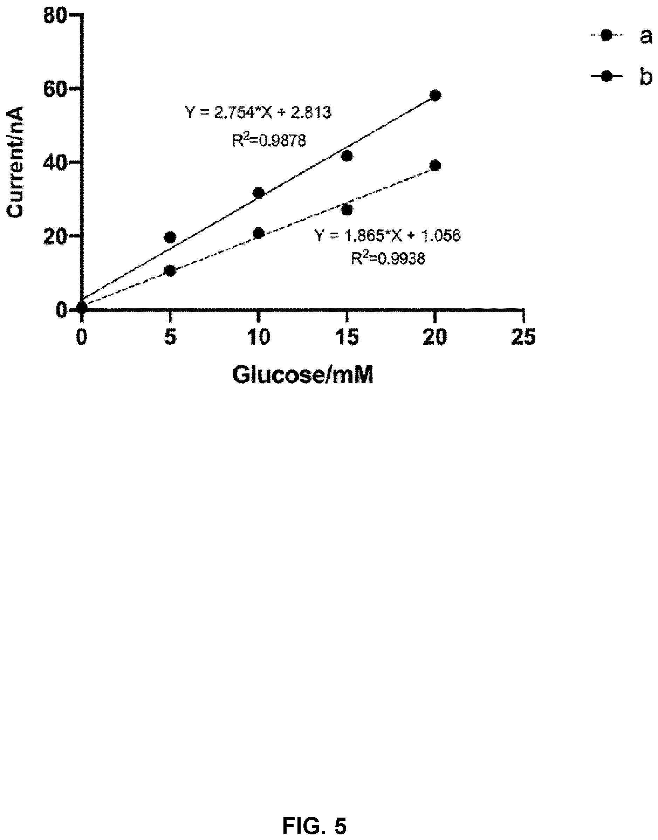

Electrochemical performance was tested in glucose buffers of varying concentrations. Results in Figures 4 to 5 show: Figure 4, Current response of glucose oxidase on the biosensor versus glucose concentration. Figure 5, Linear correlation between current and glucose concentration.

Data indicate that the deglycosylated glucose oxidase exhibits faster electron transfer, leading to higher current output and sensitivity in biosensors at equivalent glucose concentrations, confirming improved electron transfer ability.

### Example 2

The preparation methods for the glucose oxidase mutant in Example 2 are substantially similar to those in Example 1, with the exception that the construction of the recombinant mutant expression plasmid pMJB1-GOx-deglycosylation differs in the mutation primers used. The specific steps are as follows:

3. Construction of Recombinant Mutant Expression Plasmid pMJB1-GOx-deglycosylation

Using the pMJB1-GOx plasmid as a template, asparagine residues at positions N43, N89, N161, N165, N258, N355, N388, and N473 were mutated to alanine (A) using the point mutation primers listed in Table 7.

**Table 7**

| | **Forward Primers (5'-3')** | **Reverse Primers (5'-3')** |
|---|---|---|
| N43A | AGAACCCCGCCATCAGTGT GCTCGTCATCGAAA (SEQ ID NO:29) | ACTGATGGCGGGGTTCTCCGT CAGACGAGCAG (SEQ ID NO:30) |
| N89A | TACCAACGCCCAAACCGCG CTGATCCGCTCCG (SEQ ID NO:31) | CGGTTTGGGCGTTGGTAGCGA GCTCCACGGTC (SEQ ID NO:32) |
| N161A | CTACTTCGCCGCATCCTGCC ATGGTGTTAATG (SEQ ID NO:33) | AGGATGCGGCGAAGTAGTGG CCAGCAGCGATC (SEQ ID NO:34) |
| N165A | ATGGTGTTGCCGGTACTGTC CATGCCGGACCC (SEQ ID NO:35) | AGTACCGGCAACACCATGGCA GGATGCGTTGA (SEQ ID NO:36) |
| N258A | TGCTCCTTAGCCAGGCCGG CACCACCCCTCGTGCC (SEQ ID NO:37) | GGCCTGGCTAAGGAGCACCTT ACCAACATATT (SEQ ID NO:38) |
| N355A | ACCTTCGCCGAGACCTTTGG TGACTATTCCGA (SEQ ID NO:39) | AAGGTCTCGGCGAAGGTGGC GAACCAAGCGGC (SEQ ID NO:40) |
| N388A | ATTCCACGCCACCACCGCCT TGCTCATCCAGT (SEQ ID | CGGTGGTGGCGTGGAATCCG CCACGGGCGACG (SEQ ID |
| | NO:41) | NO:42) |
| N473A | CCGCGCCATCTCCAACTCC GGTGCCATGCAGA (SEQ ID NO:43) | AGTTGGAGATGGCGCGGGCC AGTTGAGTAGCG (SEQ ID NO:44) |

### 5. Redox Potential Detection

Ferrocenyl methanol was used as an electron mediator for the wild-type/deglycosylated glucose oxidase mutants obtained above.

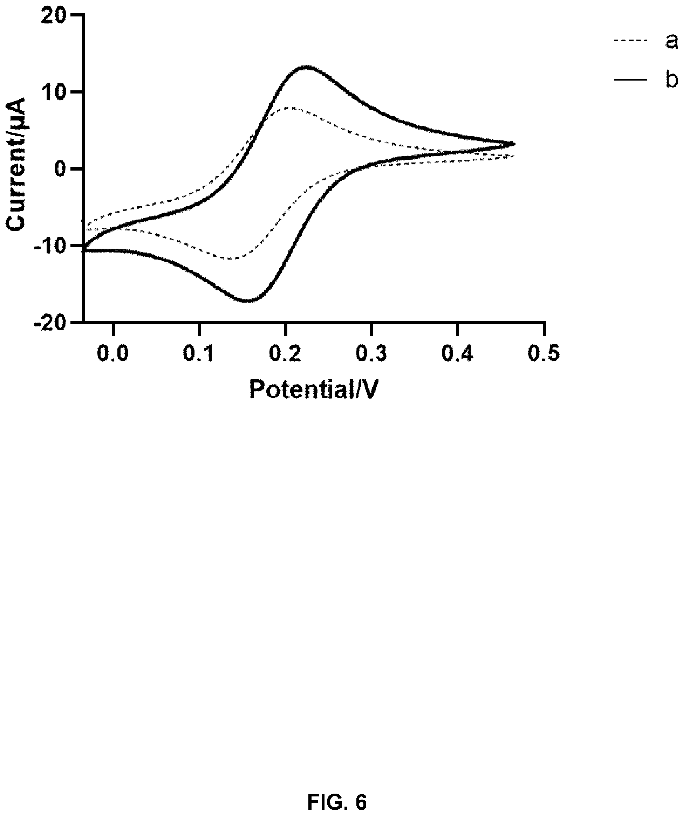

Polish and clean a glassy carbon electrode via ultrasonic cleaning.Apply 5 µL of ferrocenyl methanol solution to the electrode surface, followed by dropwise addition of wild-type/mutant glucose oxidase pure enzyme solution. Cure the electrode in a glutaraldehyde vapor environment for 30 minutes, then dry overnight at 45°C.Measure the redox potential using an Ag/AgCl reference electrode and a platinum counter electrode in a phosphate buffer system with an electrochemical workstation, as shown in Figure 6.

### Example 3

### 1. Preparation of Recombinant Expression Plasmid pMJB1 - CAT

Using the human genome as a template, the catalase gene (CAT) was amplified by PCR using primer sequences 5'- ATGGCTGACAGCCGGGATCC - 3'(SEQ ID NO:45) and 5'- TCACAGATTTGCCTTCTCCC - 3'(SEQ ID NO:46). The target bands and non - target bands were separated by DNA agarose gel electrophoresis, and the catalase gene solution was obtained after gel extraction.

A linearized forward primer 5'- TATCCATACGATGTTCCTGATTATGC - 3'(SEQ ID NO: 47) and a reverse primer 5'-ATACTGTTTAAATTAATCTATGCTAGCTTATTT- 3'(SEQ ID NO: 48) were designed. The pMJB1 vector was linearized using the homologous recombination method.

The plasmid was linearized by polymerase chain reaction (PCR) using a DNA polymerase such as the commercialized PrimerSTAR Max Premix (2X). The PCR reaction solution was prepared as shown in Table 1, and the PCR reaction was carried out under the conditions shown in Table 2. After the PCR reaction, the reaction solution was subjected to DNA agarose gel electrophoresis, and the gel was extracted to obtain the linearized pMJB1 vector.

### 2. Construction of Recombinant Expression Plasmid pMJB1 - CAT

The recovered linearized pMJB1 vector was ligated to the catalase gene fragment using a homologous recombinase. The reaction setup is shown in Table 8. The prepared reaction solution was incubated at 37°C for 30 min and then immediately transferred to 4°C for cooling and storage.

**Table 8**

| **Reagents** | **Amount Used (µL)** |
|---|---|
| 5X CE II Buffer | 4 |
| Exnase II | 2 |
| Catalase gene | 4 |
| pMJB1 linearized template | 2 |
| Sterilized Water | 8 |

The pMJB1 - CAT recombinant products were added to DH5α competent cells thawed on ice, mixed, and incubated on ice for 30 min. Then, they were transferred to a 42°C water bath for 45 s, followed by incubation on ice for 2 - 3 min. Next, 1 mL of non - resistant LB liquid medium was added, and the cells were incubated with shaking at 37°C for 1 h. After centrifugation at 2000 rpm, the supernatant was removed. The remaining medium was used to resuspend the cell pellet, and the cell suspension was spread onto an LB plate medium containing ampicillin resistance using a spreading rod. The plate was incubated overnight at 37°C and then sent for sequencing. The successfully ligated pMJB1 - CAT expression plasmid was obtained.

### 3. Construction of Recombinant Mutant Expression Plasmid pMJB1 - CAT - deglycosylation

Using the pMJB1 - CAT plasmid as a template, the asparagine residues at positions N148, N244, N439, and N481 were mutated to glutamine (Q). The point - mutation primers are shown in Table 9.

**Table 9**

| | **Forward Primers (5'-3')** | **Reverse Primers (5'-3')** |
|---|---|---|
| N14 8Q | CGTTGGACAAAACACCCCCAT TTTCTTCATCA (SEQ ID NO:49) | GGGTGTTTTGTCCAACGAGATCC CAGTTACCA (SEQ ID NO:50) |
| N24 4Q | CAGGGCATCAAACAACTTTCT GTTGAAGATGCGGCG (SEQ ID NO:51) | AGTTGTTTGATGCCCTGGTCAGT CTTATAATG (SEQ ID NO:52) |
| N43 9Q | TCAAGTTACTCAGGTGCGGGC ATTCTATGTGA (SEQ ID NO:53) | GCACCTGAGTAACTTGATCATCA TTGGCAGTGTTGAATCT (SEQ ID NO:54) |
| N48 1Q | [?] GGTCAAGCAATTCACTGAGGT CCACCCTGACT (SEQ ID NO:55) | CAGTGAATTGCTTGACCGCTTTC TTCTGGATG (SEQ ID NO:56) |

Point - mutation PCR was performed using PrimerStar Max Premix (2X). The preparation of the PCR reaction solution is shown in Table 5. The solution was aliquoted into a 0.2 mL PCR tube and placed in a PCR machine. The reaction conditions are shown in Table 6.

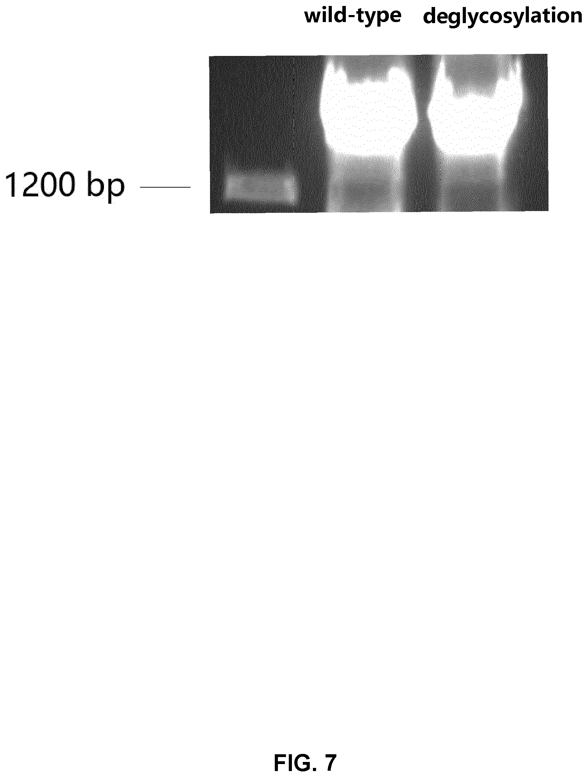

After the PCR reaction, 1 µL of DPN1 restriction endonuclease was added to the reaction mixture. After incubation at 37°C for 1 h, the PCR products were cooled to 4°C, added to DH5α competent cells thawed on ice, mixed, and incubated on ice for 30 min. Then, they were transferred to a 42°C water bath for 45 s, followed by incubation on ice for 2 - 3 min. 1 mL of non - resistant LB liquid medium was added, and the cells were incubated with shaking at 37°C for 1 h. After centrifugation at 2000 rpm, the supernatant was removed. The remaining medium was used to resuspend the cell pellet, and the cell suspension was spread onto an LB plate medium containing ampicillin resistance using a spreading rod. The plate was incubated overnight at 37°C and then sent for sequencing. The pMJB1 - CAT - deglycosylation expression plasmid was obtained. Wild - type and deglycosylated catalase DNA fragments were obtained by DNA agarose gel electrophoresis, as shown in Figure 7.

### 4. Obtaining Wild - type Catalase and Mutant Catalase

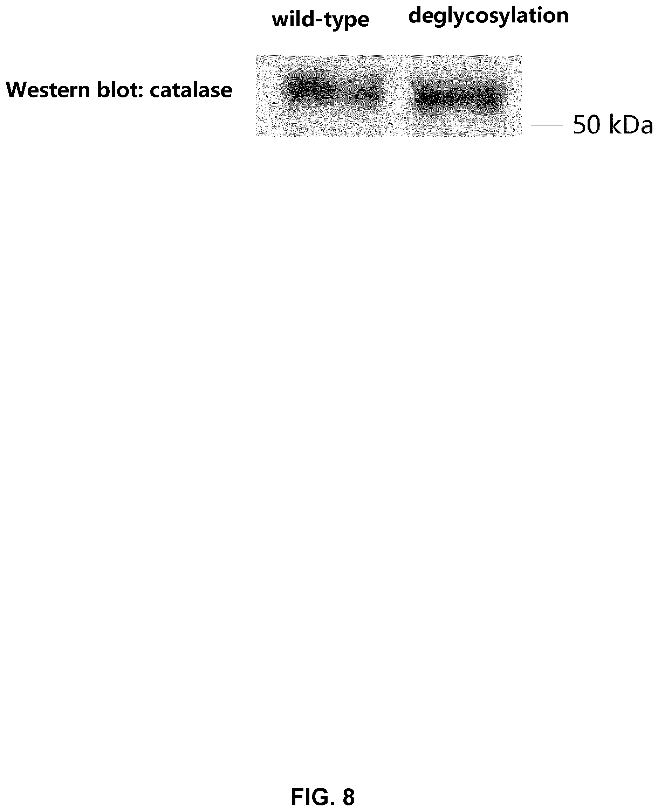

The constructed expression plasmid was transformed into *Agrobacterium* AGL1 using the freeze - thaw method. The correct *Agrobacterium* transformants were selected and cultured in liquid YEB medium for activation. 50 µL each of the activated *Agrobacterium* AGL1 and the mycelium of *Aspergillus niger* with high glucoamylasesucrase yield cultured in liquid potato medium were pipetted and evenly spread onto a PDA plate containing 200 µmol/L acetosyringone (AS) covered with a layer of cellophane. The plate was co - cultured in a 28°C incubator for 3 days. Then, the cellophane was transferred to a PDA plate containing 250 mg/L hygromycin B and 400 mg/L cefotaxime sodium for 1 day. After removing the cellophane, the culture was continued at 32°C for 7 - 10 days until the growth of Aspergillus niger was obvious. Aspergillus niger was fermented, and the fermentation products were purified. Wild - type catalase (CAT) and deglycosylated catalase (CAT - deglycosylation) mutants were obtained. The deglycosylation of catalase was verified by Western blot analysis, and the expression of deglycosylated catalase was confirmed, as shown in Figure 8.

### 5. Redox Potential Detection

Ferrocenyl methanol was used as an electron mediator in combination with the wild - type/deglycosylated catalase obtained above.

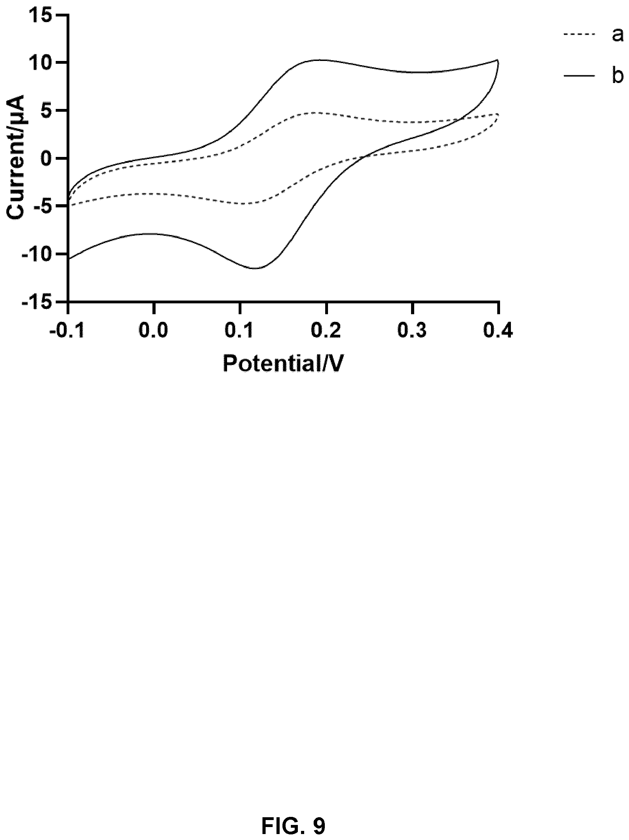

The specific steps are as follows: After a glassy carbon electrode was polished and ultrasonically cleaned, 5 µL of ferrocenyl methanol solution was added to the surface of the glassy carbon electrode. Then, the wild - type catalase and mutant pure enzyme solutions were added dropwise to the electrode surface. The electrode was then cured in a glutaraldehyde vapor atmosphere for 30 min and dried in an oven at 45°C overnight. After immobilization, the redox potential of the mixture was measured using an Ag/AgCl reference electrode and a platinum counter - electrode in a phosphate buffer system with an electrochemical workstation, as shown in Figure 9.

6. Testing the Electrochemical Performance in Hydrogen Peroxide Buffers of Different Concentrations

A conductive paste was screen - printed layer by layer on a substrate and cured to obtain an electrode pattern. An insulating medium paste was printed between the layers. Finally, the substrate was laser - cut and cleaned to obtain the substrate electrode. The working electrode and the counter - electrode of the substrate electrode were made of platinum carbon paste, and the reference electrode layer was made of silver/silver chloride paste. The wild - type/deglycosylated catalase obtained above was dissolved in a BSA solution. The substrate electrode - cobalt ion complex was immersed in a BSA solution containing catalase and glutaraldehyde for 10 min and then left to stand at room temperature for 1 h to obtain the catalase - coated sensing electrode. The electrochemical performance of the electrode was tested in buffer solutions containing different concentrations of hydrogen peroxide.

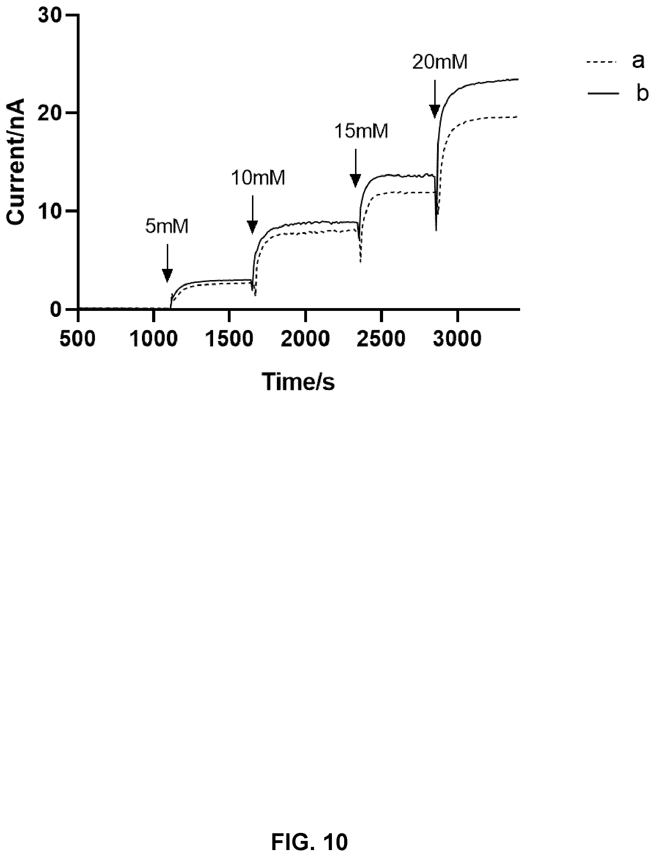

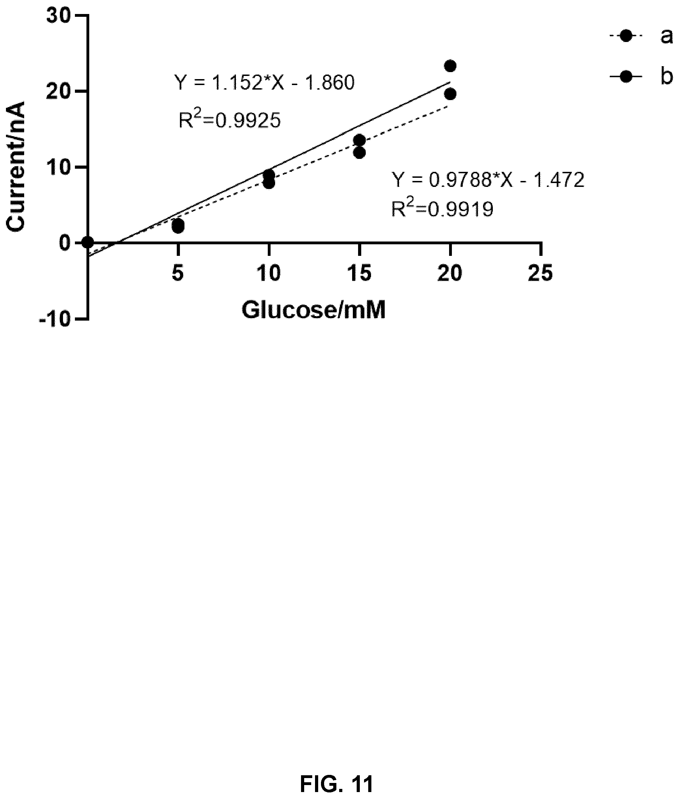

The results are shown in Figures 10 - 11. Figure 10 shows the relationship between the current of catalase on the electrochemical sensor and the concentration of hydrogen peroxide, and Figure 11 shows the linear correlation between the current of catalase on the electrochemical sensor and the concentration of hydrogen peroxide.

The above data show that deglycosylated catalase has a faster electron - transfer ability. When applied to a bioelectrochemical sensor with the same concentration of hydrogen peroxide, the bioenzyme electrode coated with deglycosylated catalase has a higher current level and a higher sensitivity level, indicating that deglycosylated catalase has a better electron - transfer ability.

### Example 4(Proportional Example 1)

The preparation methods for the glucose oxidase mutant in Proportional Example 1 are substantially similar to those in Example 1, with the exception that the construction of the recombinant mutant expression plasmid pMJB1-GOx-deglycosylation utilizes different mutation primers. The specific steps are as follows:

### 3. Construction of Recombinant Mutant Expression Plasmid pMJB1-GOx-deglycosylation

Using the pMJB1-GOx plasmid as a template, the asparagine residue at position N43 was mutated to glutamine (Q) using the point mutation primers listed in Table 10.

**Table 10**

| | **Forward Primers (5'-3')** | **Reverse Primers (5'-3')** |
|---|---|---|
| N4 3Q | GAACCCCCAATCAGTGTGCTCG TCATCGAAAG (SEQ ID NO:57) | ACACTGATTGGGGGTTCTCCGT CAGACGAGCA (SEQ ID NO:58) |

### 5. Redox Potential Detection

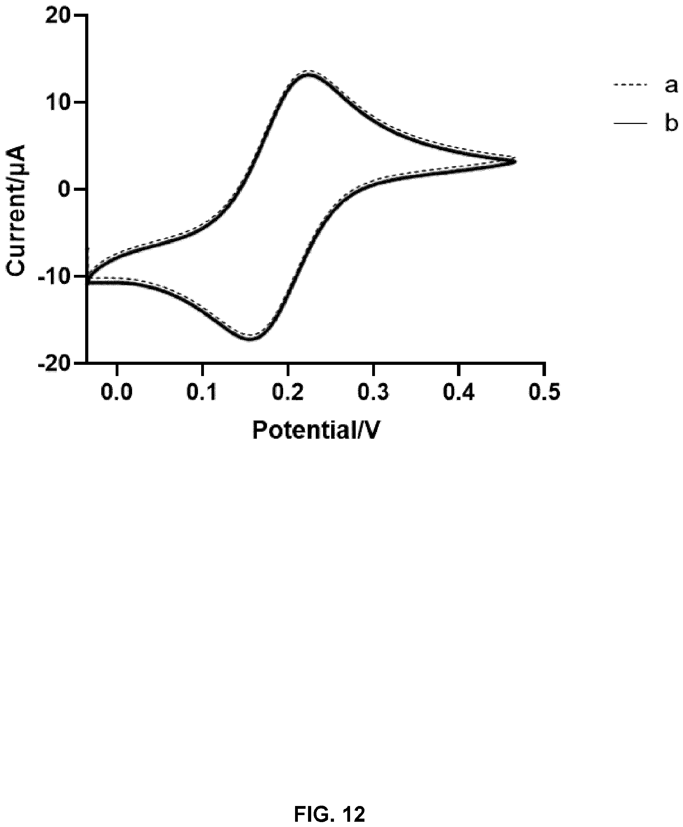

Ferrocenyl methanol was used as an electron mediator for the wild-type/mutant glucose oxidase obtained above. Polish and clean a glassy carbon electrode via ultrasonic cleaning. Apply 5 µL of ferrocenyl methanol solution to the electrode surface, followed by dropwise addition of wild-type/mutant glucose oxidase pure enzyme solution. Cure the electrode in a glutaraldehyde vapor atmosphere for 30 minutes, then dry overnight at 45°C.Measure the redox potential using an Ag/AgCl reference electrode and a platinum counter electrode in a phosphate buffer system with an electrochemical workstation, as shown in FIG. 12.The amino acid sequence of the mutant glucose oxidase is set forth as:

MQTLLVSSLVVSLAAALPHYIRSNGIEASLLTDPKDVSGRTVDYIIAGGGLTGL
TTAARLTENPQISVLVIESGSYESDRGPIIEDLNAYGDIFGSSVDHAYETVELA
TNNQTALIRSGNGLGGSTLVNGGTWTRPHKAQVDSWETVFGNEGWNWDN
VAAYSLQAERARAPNAKQIAAGHYFNASCHGVNGTVHAGPRDTGDDYSPIV
KALMSAVEDRGVPTKKDFGCGDPHGVSMFPNTLHEDQVRSDAAREWLLPN
YQRPNLQVLTGQYVGKVLLSQNGTTPRAVGVEFGTHKGNTHNVYAKHEVLL
AAGSAVSPTILEYSGIGMKSILEPLGIDTVVDLPVGLNLQDQTTATVRSRITSA
GAGQGQAAWFATFNETFGDYSEKAHELLNTKLEQWAEEAVARGGFHNTTA
LLIQYENYRDWIVNHNVAYSELFLDTAGVASFDVWDLLPFTRGYVHILDKDPY
LHHFAYDPQYFLNELDLLGQAAATQLARNISNSGAMQTYFAGETIPGDNLAY
DADLSAWTEYIPYHFRPNYHGVGTCSMMPKEMGGVVDNAARVYGVQGLRV
IDGSIPPTQMSSHVMTVFYAMALKISDAILEDYASMQ (SEQ ID NO:85) .

### Example 5(Proportional Example 2)

The preparation methods for the glucose oxidase mutant in Proportional Example 2 are substantially similar to those in Example 1. The difference lies in the fact that the mutation primers used for the construction of the recombinant mutant expression plasmid pMJB1 - GOx - deglycosylation are different. The specific steps are as follows:

### 3. Construction of the Recombinant Mutant Expression Plasmid pMJB1 - GOx - deglycosylation

Using the pMJB1 - GOx plasmid as a template, the glycine residues at positions G25, G81, G119, G152, G191, G248, G320, and G514 were mutated to alanine (A). The point - mutation primers are shown in Table 11.

**Table 11**

| | **Forward Primers (5'-3')** | **Reverse Primers (5'-3')** |
|---|---|---|
| G25 A | GAGCAATGCCATTGAAGCCAGC CTCCTGACTG (SEQ ID NO:59) | CTTCAATGGCATTGCTCCTGAT GTAGTGTGGCA (SEQ ID NO:60) |
| G81 A | GACAGAGCCCCTATCATTGAGG ACCTGAACGC (SEQ ID NO:61) | ATGATAGGGGCTCTGTCCGAC TCGTAGGAGCC (SEQ ID NO:62) |
| G11 9A | TGATCCGCTCCGCCAATGGTCT CGGTGGCTCTACTC (SEQ ID NO:63) | ATTGGCGGAGCGGATCAGCG CGGTTTGATTGT (SEQ ID NO:64) |
| G15 2A | AAATGAGGCCTGGAACTGGGAC AATGTGGCCG (SEQ ID NO:65) | AGTTCCAGGCCTCATTTCCAAA GACAGTCTCCCA (SEQ ID NO:66) |
| G19 1A | TGTTAATGCCACTGTCCATGCCG GACCCCGCG (SEQ ID NO:67) | GGACAGTGGCATTAACACCAT GGCAGGATGCG (SEQ ID |
| | | NO:68) |
| G24 8A | ATATGTTGCCAAGGTGCTCCTTA GCCAGAACG (SEQ ID NO:69) | GCACCTTGGCAACATATTGGT CTTCGTGCAAGG (SEQ ID NO:70) |
| G32 0A | GTCGCCTTGAACCTGCAGGACC AGACCACCGC (SEQ ID NO:71) | TGCAGGTTCAAGGCGACGGG CAGGTCAACGACG (SEQ ID NO:72) |
| G51 4A | TAACTACCATGCCGTGGGTACTT GCTCCATGATGC (SEQ ID NO:73) | CCACGGCATGGTAGTTAGGAC GGAAGTGGTAC (SEQ ID NO:74) |

### 5. Redox Potential Detection

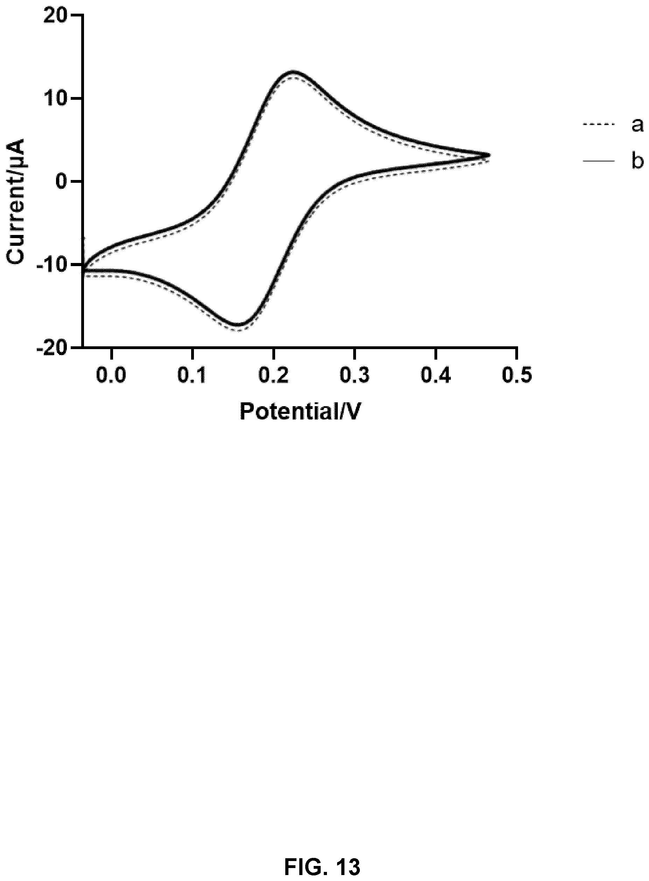

Ferrocenyl methanol was used as an electron mediator in combination with the wild - type/mutant glucose oxidase obtained above. A glassy carbon electrode was polished and ultrasonically cleaned. Then, 5 µL of ferrocenyl methanol solution was added to the surface of the glassy carbon electrode. Subsequently, the wild - type glucose oxidase and mutant pure enzyme solutions were added dropwise to the electrode surface. The electrode was then cured in a glutaraldehyde vapor atmosphere for 30 minutes and dried in an oven at 45°C overnight. After immobilization, the redox potential of the mixture was measured using an Ag/AgCl reference electrode and a platinum counter - electrode in a phosphate buffer system with an electrochemical workstation, as shown in Figure 13. The amino acid sequence of the mutant glucose oxidase is as follows:

MQTLLVSSLVVSLAAALPHYIRSNAIEASLLTDPKDVSGRTVDYIIAGGGLTGL
TTAARLTENPNISVLVIESGSYESDRAPIIEDLNAYGDIFGSSVDHAYETVELAT
NNQTALIRSANGLGGSTLVNGGTWTRPHKAQVDSWETVFGNEAWNWDNV

AAYSLQAERARAPNAKQIAAGHYFNASCHGVNATVHAGPRDTGDDYSPIVK
ALMSAVEDRGVPTKKDFGCGDPHGVSMFPNTLHEDQYVAKVLLSQNGTTP
RAVGVEFGTHKGNTHNVYAKHEVLLAAGSAVSPTILEYSGIGMKSILEPLGID
TVVDLPVALNLQDQTTATVRSRITSAGAGQGQAAWFATFNETFGDYSEKAH
ELLNTKLEQWAEEAVARGGFHNTTALLIQYENYRDWIVNHNVAYSELFLDTA
GVASFDVWDLLPFTRGYVHILDKDPYLHHFAYDPQYFLNELDLLGQAAATQL
ARNISNSGAMQTYFAGETIPGDNLAYDADLSAWTEYIPYHFRPNYHAVGTCS
MMPKEMGGVVDNAARVYGVQGLRVIDGSIPPTQMSSHVMTVFYAMALKISD
AILEDYASMQ (SEQ ID NO: 86).

### Example 6(Proportional Example 3)

The preparation methods for the catalase mutants in Proportional Example 3 are substantially similar to those in Example 3. The difference is that the mutation primers used for the construction of the recombinant mutant expression plasmid pMJB1 - CAT - deglycosylation are different. The specific steps are as follows:

### 3. Construction of the Recombinant Mutant Expression Plasmid pMJB1 - CAT - deglycosylation

Using the pMJB1 - CAT plasmid as a template, the asparagine residue at position N148 was mutated to glutamine (Q). The point - mutation primers are shown in Table 12.

**Table 12**

| | **Forward Primers (5'-3')** | **Reverse Primers (5'-3')** |
|---|---|---|
| N14 8Q | CGTTGGACAAAACACCCCCATT TTCTTCATCA (SEQ ID NO:75) | GGGTGTTTTGTCCAACGAGATC CCAGTTACCA (SEQ ID NO:76) |

### 5. Redox Potential Detection

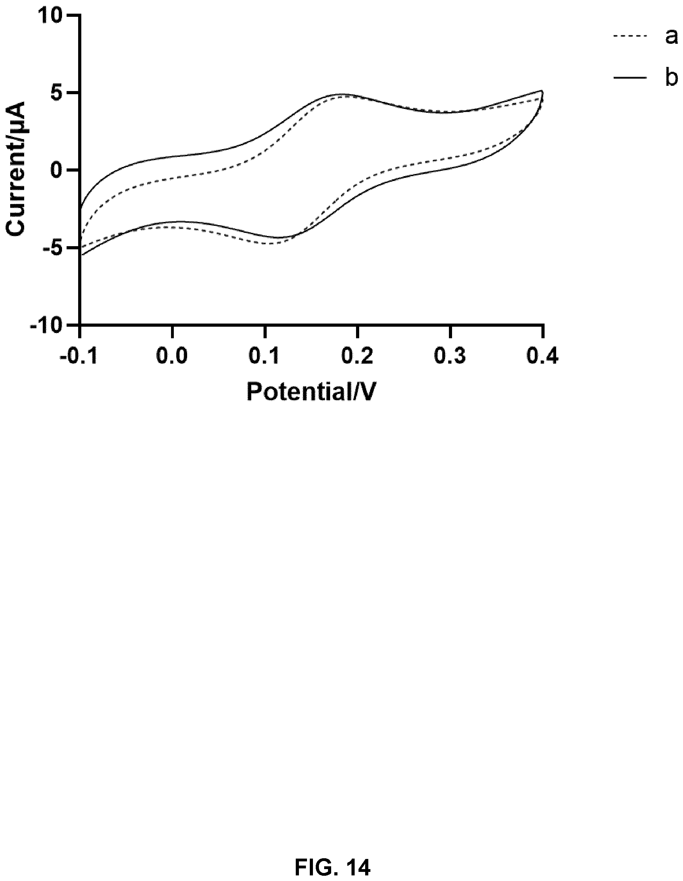

Ferrocenyl methanol was used as an electron mediator in combination with the wild - type/mutant catalase obtained above. A glassy carbon electrode was polished and ultrasonically cleaned. Then, 5 µL of ferrocenyl methanol solution was added to the surface of the glassy carbon electrode and allowed to air - dry. Subsequently, the wild - type catalase and mutant pure enzyme solutions were added to the electrode surface and also allowed to air - dry. The electrode was then cured in a glutaraldehyde vapor environment for 30 minutes and dried in an oven at 45°C overnight. After immobilization, the redox potential of the mixture was measured using an Ag/AgCl reference electrode and a platinum counter - electrode in a phosphate buffer system with an electrochemical workstation, as shown in FIG. 14. The amino acid sequence of the mutant catalase is as follows:

MADSRDPASDQMQHWKEQRAAQKADVLTTGAGNPVGDKLNVITVGPRGPL
LVQDVVFTDEMAHFDRERIPERVVHAKGAGAFGYFEVTHDITKYSKAKVFEHI
GKKTPIAVRFSTVAGESGSADTVRDPRGFAVKFYTEDGNWDLVGQNTPIFFI
RDPILFPSFIHSQKRNPQTHLKDPDMVWDFWSLRPESLHQVSFLFSDRGIPD
GHRHMNGYGSHTFKLVNANGEAVYCKFHYKTDQGIKNLSVEDAARLSQEDP
DYGIRDLFNAIATGKYPSWTFYIQVMTFNQAETFPFNPFDLTKVWPHKDYPLI
PVGKLVLNRNPVNYFAEVEQIAFDPSNMPPGIEASPDKMLQGRLFAYPDTHR
HRLGPNYLHIPVNCPYRARVANYQRDGPMCMQDNQGGAPNYYPNSFGAPE
QQPSALEHSIQYSGEVRRFNTANDDNVTQVRAFYVNVLNEEQRKRLCENIA
GHLKDAQIFIQKKAVKNFTEVHPDYGSHIQALLDKYNAEKPKNAIHTFVQSGS
HLAAREKANL (SEQ ID NO: 87).

### Example 7(Proportional Example 4)

The preparation methods for the catalase mutants in Proportional Example 4 are substantially similar to those in Example 3. The difference is that the mutation primers used for the construction of the recombinant mutant expression plasmid pMJB1 - CAT - deglycosylation are different. The specific steps are as follows:

### 3. Construction of the Recombinant Mutant Expression Plasmid pMJB1 - CAT - deglycosylation

Using the pMJB1 - CAT plasmid as a template, the glycine residues at positions G78, G204, G272, and G465 were mutated to alanine (A). The point - mutation primers are shown in Table 13.

**Table 13**

| | **Forward Primers (5'-3')** | **Reverse Primers (5'-3')** |
|---|---|---|
| G78 A | TGCATGCTAAAGCCGCAGGGG CCTTTGGCTAC (SEQ ID NO:77) | TGCGGCTTTAGCATGCACAACT CTCTCAGGAA (SEQ ID NO:78) |
| G20 4A | ATCGGGCCATTCCAGATGGACA TCGCCACATG (SEQ ID NO:79) | ATCTGGAATGGCCCGATCACTG AACAAGAAAGAAAC (SEQ ID NO:80) |
| G27 2A | TTGCCACAGCCAAGTACCCCTC CTGGACTTTTTACA (SEQ ID NO:81) | GTACTTGGCTGTGGCAATGGCG TTAAAAAGAT (SEQ ID NO:82) |
| G46 5A | ATTGCCGCCCACCTGAAGGAT GCACAAATTTT (SEQ ID NO:83) | TTCAGGTGGGCGGCAATGTTCT CACACAGACGTT (SEQ ID NO:84) |

### 5. Redox Potential Detection

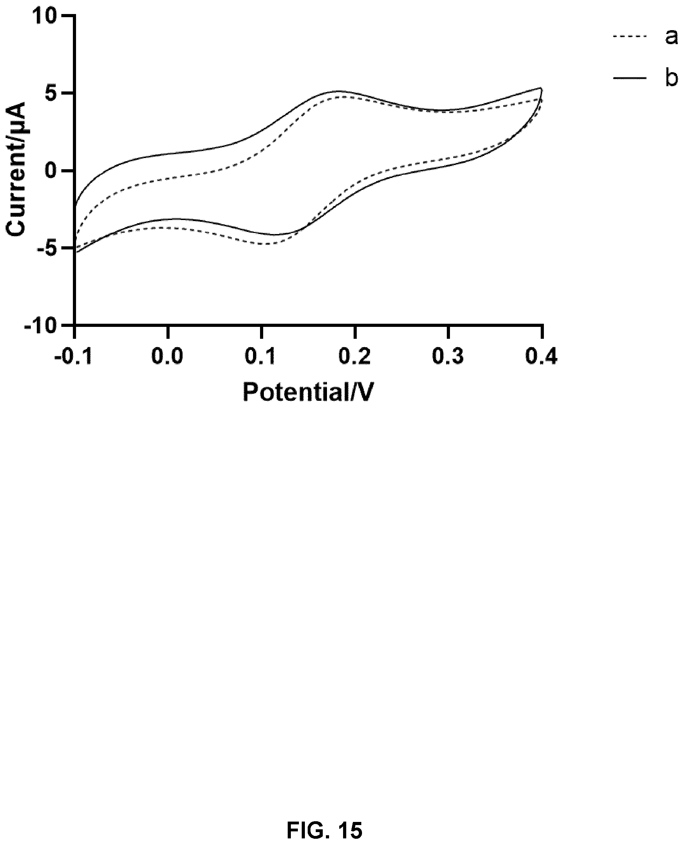

Ferrocenyl methanol was used as an electron mediator in combination with the wild - type/mutant catalase obtained above. A glassy carbon electrode was polished and ultrasonically cleaned. Then, 5 µL of ferrocenyl methanol solution was added to the surface of the glassy carbon electrode. Subsequently, the wild - type catalase and mutant pure enzyme solutions were added dropwise to the electrode surface. The electrode was then cured in a glutaraldehyde vapor environment for 30 minutes and dried in an oven at 45°C overnight. After immobilization, the redox potential of the mixture was measured using an Ag/AgCl reference electrode and a platinum counter - electrode in a phosphate buffer system with an electrochemical workstation, as shown in FIG. 15. The amino acid sequence of the mutant catalase is as follows:

MADSRDPASDQMQHWKEQRAAQKADVLTTGAGNPVGDKLNVITVGPRGPL
LVQDVVFTDEMAHFDRERIPERVVHAKAAGAFGYFEVTHDITKYSKAKVFEHI
GKKTPIAVRFSTVAGESGSADTVRDPRGFAVKFYTEDGNWDLVGNNTPIFFI
RDPILFPSFIHSQKRNPQTHLKDPDMVWDFWSLRPESLHQVSFLFSDRAIPD
GHRHMNGYGSHTFKLVNANGEAVYCKFHYKTDQGIKNLSVEDAARLSQEDP

DYGIRDLFNAIATAKYPSWTFYIQVMTFNQAETFPFNPFDLTKVWPHKDYPLI
PVGKLVLNRNPVNYFAEVEQIAFDPSNMPPGIEASPDKMLQGRLFAYPDTHR
HRLGPNYLHIPVNCPYRARVANYQRDGPMCMQDNQGGAPNYYPNSFGAPE
QQPSALEHSIQYSGEVRRFNTANDDNVTQVRAFYVNVLNEEQRKRLCENIAA
HLKDAQIFIQKKAVKNFTEVHPDYGSHIQALLDKYNAEKPKNAIHTFVQSGSH
LAAREKANL (SEQ ID NO: 88).

In summary, the biological enzyme mutants prepared in the embodiments of this application can be used to fabricate a biosensor through direct electron transfer between the biological enzyme and the surface of the substrate electrode. This results in a higher current level and a higher sensitivity level compared to the corresponding wild - type enzyme or other reference ratios.

The various technical features of the above embodiments may be arbitrarily combined. For brevity, not all possible combinations of the technical features described in the foregoing embodiments are explicitly recited. Nevertheless, any combination of these technical features that does not create a conflict shall be deemed to fall within the scope of this specification.

The above embodiments merely describe several implementations of this application. While their descriptions are specific and detailed, they should not be construed as limiting the scope of the patent application. It is noted that those of ordinary skill in the art may make various modifications and improvements without departing from the spirit and scope of this application, and such modifications and improvements are intended to be within the scope of protection hereof. Accordingly, the scope of protection for this patent application is defined by the appended claims.

## Claims

1. A biological enzyme mutant, comprising at least one of a glucose oxidase mutant and a catalase mutant, wherein:
the glucose oxidase mutant has, relative to the wild-type glucose oxidase, at least one of the following sets of mutations:
(i) p.N43Q, p.N89Q, p.N161Q, p.N165Q, p.N258Q, p.N355Q, p.N388Q, and p.N473Q; or
(ii) p.N43A, p.N89A, p.N161A, p.N165A, p.N258A, p.N355A, p.N388A, and p.N473A;
when the biological enzyme mutant comprises a catalase mutant, the catalase mutant has, relative to the wild-type catalase, the mutations p.N148Q, p.N244Q, p.N439Q, and p.N481Q;
the amino acid sequence of the wild-type glucose oxidase is set forth in SEQ ID NO:1, and the amino acid sequence of the wild-type catalase is set forth in SEQ ID NO:2.

2. The biological enzyme mutant of claim 1, wherein the amino acid sequence of the glucose oxidase mutant is set forth in SEQ ID NO:3 or SEQ ID NO:4, and/or the amino acid sequence of the catalase mutant is set forth in SEQ ID NO:5.

3. A nucleic acid molecule encoding the biological enzyme mutant of claim 1 or 2.

4. A recombinant vector comprising the nucleic acid molecule of claim 3.

5. The recombinant vector of claim 4, wherein the vector used for expressing the enzyme mutant is pMJB1.

6. A host cell comprising the recombinant vector of claim 4 or 5 in its genome.

7. The host cell of claim 6, wherein the host cell is the recipient cell.

8. The host cell of claim 7, wherein the recipient cell is selected from the group comprising *Escherichia coli, Agrobacterium, Saccharomyces cerevisiae, Pichia pastoris, Aspergillus niger,* an animal cell, and a plant cell.

9. The host cell of claim 7, wherein the recipient cell is selected from the group comprising *Escherichia coli* DH5α, *Escherichia coli* Top10, *Escherichia coli* Origami(DE3), *Agrobacterium* AGL1, *Aspergillus niger, Pichia pastoris* GS115, or *Pichia pastoris* SMD1168.

10. Use of the biological enzyme mutant of claim 1 or 2, the nucleic acid molecule of claim 3, the recombinant vector of claim 4 or 5, or the host cell of any one of claims 6 to 9 in the preparation of a biological enzyme.

11. A method for preparing the biological enzyme mutant of claim 1 or 2, the method comprising:
introducing mutations into at least one of (i) the wild-type glucose oxidase having the amino acid sequence of SEQ ID NO:1 or (ii) the wild-type catalase having the amino acid sequence of SEQ ID NO:2, wherein:
for the glucose oxidase, introducing mutations at positions 43, 89, 161, 165, 258, 355, 388, and 473 to produce either (A) asparagine-to-glutamine substitutions (N→Q) or (B) asparagine-to-alanine substitutions (N→A); and
when introducing mutations into the wild-type catalase, for the catalase, introducing mutations at positions 148, 244, 439, and 481 to produce asparagine-to-glutamine substitutions (N→Q).

12. The method of claim 11, further comprising:
(a) preparing a first linear recombinant vector comprising a glucose oxidase gene and a second linear recombinant vector comprising a catalase gene;
(b) performing a first PCR amplification on the first linear recombinant vector using primers set forth in SEQ ID NO:13-28 and SEQ ID NO:29-44 to obtain first and second mutant recombinant vectors; performing a second PCR amplification on the second linear recombinant vector using primers set forth in SEQ ID NO:49-56 to obtain a third mutant recombinant vector; and
(c) transforming the first, second, and third mutant recombinant vectors into a host cell and culturing the host cell to produce the biological enzyme mutant.

13. Use of the biological enzyme mutant of claim 1 in the preparation of a biosensing element, wherein the biosensing element comprises a bioenzyme electrode or a biosensor.

14. A bioenzyme electrode, comprising:
a base electrode; and
a modification material supported on the base electrode, wherein the modification material comprises the biological enzyme mutant of claim 1.

15. A biosensor comprising the bioenzyme electrode of claim 14.
